# FASCICULE DE BREVET EUROPEEN

(11) **EP 0 552 282 B1**
(45) Date de publication et mention de la délivrance du brevet: **24.08.1994**
(21) Numéro de dépôt: 91919625.3
(22) Date de dépôt: 11.10.1991
(51) Int. Cl.: C07C 69/86, C07C 69/76, C07C 233/76, A61K 31/215, A61K 31/19, A61K 7/00, C07C 327/20, C07C 69/96

(54) **COMPOSES BI-AROMATIQUES ET LEUR UTILISATION EN MEDECINE HUMAINE ET VETERINAIRE ET EN COSMETIQUE**
BIAROMATISCHE VERBINDUNGEN UND IHRE ANWENDUNG IN MENSCHEN- UND TIERHEILKUNDE UND IN DER KOSMETIK
BI-AROMATIC COMPOUNDS AND THEIR USE IN HUMAN AND VETERINARY MEDECINE AND IN COSMETIC PREPARATIONS

(30) Priorité: 12.10.1990 LU 87821
(43) Date de publication de la demande: 28.07.1993
(73) Titulaire: CENTRE INTERNATIONAL DE RECHERCHES DERMATOLOGIQUES GALDERMA - CIRD GALDERMA, F-06565 Valbonne (FR)
(72) Inventeur: BERNARDON, Jean-Michel 21, chemin Plan-Bergier, F-06650 Nice (FR); PILGRIM, William, Robert, F-06560 Valbonne (FR)
(74) Mandataire: Stalla-Bourdillon, Bernard
(86) Numéro de dépôt international: FR9100793
(87) Numéro de publication internationale: WO9206948

(56) Documents cités:
- DE-A- 3 903 988
- CHEMICAL ABSTRACTS, VOL. 110, NO. 3, 16 JANVIER 1989, COLUMBUS, OHIO, US; H. KAGECHIKA: "RETINOBENZOIC ACIDS.1.STRUCTURE-ACTIVITY RELATIONSHIPS OF AROMATIC AMIDES WITH RETINOIDAL ACTIVITY", VOIR P. 512, ABRéGé 23483P
- CHEMICAL ABSTRACTS, VOL. 111, NO. 15, 9 OCTOBRE 1989, COLUMBUS, OHIO, US; H. KAGECHIKA: "RETINOBENZOIC ACIDS.4.CONFORMATION OF AROMATIC AMIDES WITH RETINOIDAL ACTIVITY. IMPORTANCE OF TRANS-AMIDE STRUCTURE FOR THE ACTIVITY", VOIR P. 797, ABRéGé 134547C

## Description

La présente invention a pour objet de nouveaux composés bi-aromatiques, leur procédé de préparation et leur utilisation en médecine humaine et vétérinaire et en cosmétique.

Des composés bi-aromatiques sont déjà connus des documents DE, A, 390 39 88; J. Med. Chem. 1988, 31 (11) 2182-92 et J. Med Chem. 1989, 32 (10) 2292-6.

Ces nouveaux composés bi-aromatiques trouvent une application dans le traitement topique et systémique des affections dermatologiques liées à un désordre de la kératinisation (différenciation-prolifération) et d'affections dermatologiques, ou autres, à composantes inflammatoires et/ou immunoallergiques et dans les maladies de dégénérescence du tissu conjonctif, et présentent une activité antitumorale. En outre, ces composés peuvent être utilisés dans le traitement de l'atopie, qu'elle soit cutanée ou respiratoire et du psoriasis rhumatoïde.

Ils trouvent également une application dans le domaine ophtalmologique, notamment dans le traitement des cornéopathies.

Les composés bi-aromatiques selon l'invention peuvent être représentés par la formule générale suivante : 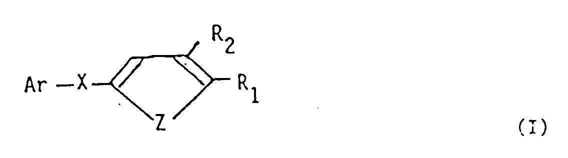
dans laquelle :
Ar représente soit le radical 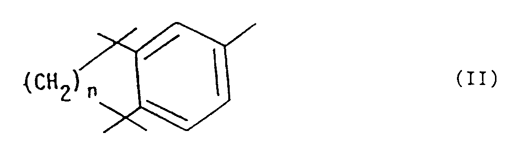
avec n = 1 ou 2
soit le radical 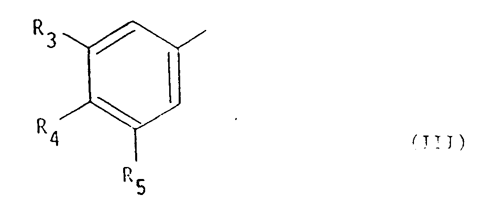
- R₃ et R₅: représentant un atome d'hydrogène, le groupe OH, un radical alcoxy ayant de 1 à 6 atomes de carbone, un radical alkyle α -substitué ayant de 3 à 12 atomes de carbone ou un radical alkyle α,α'-disubstitué ayant de 4 à 12 atomes de carbone, un radical cycloalkyle ayant de 3 à 12 atomes de carbone, un radical cycloalkyle mono ou polycyclique ayant de 5 à 12 atomes de carbone dont le carbone de liaison est trisubstitué,
- R₄: représentant un atome d'hydrogène, le groupe OH, un radical alcoxy ayant de 1 à 6 atomes de carbone, un radical alkyle α-substitué ayant de 3 à 12 atomes de carbone, un radical alkyle α,α'-disubstitué ayant de 4 à 12 atomes de carbone, un radical cycloalkyle ayant de 3 à 12 atomes de carbone, un radical cycloalkyle mono ou polycyclique ayant de 5 à 12 atomes de carbone dont le carbone de liaison est trisubstitué, un radical monohydroxyalkyle, un radical polyhydroxyalkyle, un atome de fluor, un atome de chlore, le groupe SH, le groupe SR₆, le groupe SOR₆, le groupe SO₂R₆, un radical alkényle ayant de 2 à 6 atomes de carbone, ou un radical alkényloxy ayant de 2 à 6 atomes de carbone,
- R₆: représentant un radical alkyle inférieur,
- R₁: représente un atome d'hydrogène, le groupe OH, le radical -CH₃, le radical -CH₂OH, le radical -COR₇, le radical -CH(OH)CH₃, le radical -CH₂OCOR₈, le radical -SO₂R₉, le radical -SOR₉ ou le radical -SR₉,
- R₇: représentant un atome d'hydrogène, le groupe OH, le radical -OR₁₀, le radical -N(r'r''), un radical alkyle inférieur, un radical monohydroxyalkyle, un radical polyhydroxyalkyle ou le reste d'un sucre,
- R₁₀: représentant un radical alkyle ayant de 1 à 12 atomes de carbone ou un radical alkényle ayant de 2 à 12 atomes de carbone,
- r' et r'': identiques ou différents, représentent un atome d'hydrogène, un radical alkyle inférieur, un radical aryle, un radical aralkyle, un reste d'aminoacide, un reste de sucre, un reste de sucre aminé, ou un hétérocycle, ou r' et r'' pris ensemble forment un hétérocycle,
- R₈: représentant un radical alkyle linéaire ou ramifié saturé ou insaturé ayant de 1 à 20 atomes de carbone ou le reste d'un sucre,
- R₉: représentant le groupe OH, un radical alkyle inférieur ou le radical -N(r'r''),
- R₂: représentant un atome d'hydrogène, le groupe OH, un radical alkyle inférieur, un radical alcoxy ayant de 1 à 6 atomes de carbone, un atome de fluor, un atome de chlore, le groupe CF₃, le groupe COR₇, le groupe CH₂OH ou le groupe CH₂OR₆,
Z représente un atome d'oxygène ou de soufre, le radical divalent -CH=CR₁₁-, le radical divalent -N=CH- ou le radical divalent -N=CR₆-,
- R₁₁: représentant un atome d'hydrogène, le groupe OH, un radical alkyle inférieur, un radical alcoxy ayant de 1 à 6 atomes de carbone, un atome de fluor, un atome de chlore, ou le groupe CF₃,
X est un radical divalent qui peut être lu de gauche à droite ou inversement, choisi dans le groupe constitué par :
(i) 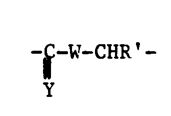 R' représentant un atome d'hydrogène, le radical -CH₃,
   W représentant un atome d'oxygène ou de soufre ou le groupe -NR',
   Y représentant un atome d'oxygène ou encore un atome de soufre lorsque W représente le groupe -NR',
(ii) 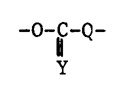 Q représentant un atome d'oxygène ou -NR',
   Y représentant un atome d'oxygène ou encore un atome de soufre lorsque Q représente, le groupe -NR', (iii) 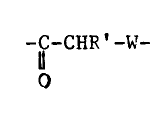 (iv) 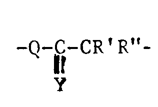 R'' représentant un atome d'hydrogène, le radical -CH₃, le groupe OH, un atome de fluor ou un atome de chlore, ou
   R' et R'' pris ensemble forment un radical méthano (=CH₂) ou un radical oxo (=O),
(v) 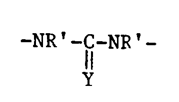 Y représentant un atome d'oxygène ou un atome de soufre,
sous réserve que lorsque simultanément X représente 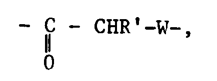
Z représente -CH = CH - et R₂ représente un atome d'hydrogène alors Ar représente le radical de formule (III) dans laquelle R₃ et R₅ sont différents d'un atome d'hydrogène ou d'un radical alkyle α-substitué ou α-α' disubstitué ayant de 3 à 5 atomes de carbone,
et les sels des composés de formule (I) lorsque R₁ représente une fonction acide carboxylique et les isomères optiques desdits composés de formule (I).

Lorsque les composés selon l'invention se présentent sous forme de sels, il s'agit alors de sels d'un métal alcalin ou alcalino-terreux ou encore de zinc ou d'une amine organique.

Par radical alkyle inférieur, on entend un radical ayant de 1 à 6 atomes de carbone et de préférence les radicaux méthyle, éthyle, isopropyle, butyle et tertiobutyle.

Par radical alcoxy ayant de 1 à 6 atomes de carbone, on doit entendre un radical méthoxy, éthoxy, isopropoxy ou butoxy.

Par radical alkyle α-substitué ayant de 3 à 12 atomes de carbone, on doit notamment entendre un radical isopropyle, 1-méthylpropyle ou 1-éthylpropyle.

Par radical alkyle α,α'-disubstitué ayant de 4 à 12 atomes de carbone, on doit notamment entendre un radical tert-butyle, 1,1-diméthyl propyle, 1-méthyl 1-éthyl propyle, 1-méthyl 1-éthyl hexyle ou 1,1-diméthyl décyle.

Par radical monohydroxyalkyle, on doit entendre un radical ayant de 1 à 6 atomes de carbone, notamment un radical 2-hydroxyéthyle, 2-hydroxypropyle ou 3-hydroxypropyle.

Par radical polyhydroxyalkyle, on doit entendre un radical contenant de 2 à 6 atomes de carbone et de 2 à 5 groupes hydroxyles tels que les radicaux 2,3-dihydroxypropyle, 2,3,4-trihydroxybutyle, 2,3,4,5-tétrahydroxypentyle ou le reste du pentaérythritol.

Par radical aryle, on doit entendre un radical phényle éventuellement substitué par au moins un atome d'halogène, un hydroxyle ou une fonction nitro.

Par radical aralkyle, on doit entendre le radical benzyle ou phénéthyle éventuellement substitué par au moins un atome d'halogène, un hydroxyle ou une fonction nitro.

Par radical cycloalkyle ayant de 3 à 12 atomes de carbone, on doit entendre notamment un radical cyclopentyle ou cyclohexyle.

Par radical cycloalkyle mono ou polycyclique ayant de 5 à 12 atomes de carbone dont le carbone de liaison est trisubstitué, on doit entendre le radical 1-méthyl cyclohexyle ou 1-adamantyle.

Par radical alkényloxy ayant de 2 à 6 atomes de carbone on doit entendre des radicaux linéaires ou ramifiés notamment allyloxy et vinyloxy.

Par radical alkényle ayant de 2 à 6 atomes de carbone, on doit entendre notamment les radicaux vinyle, allyle ou 2-butényle.

Lorsque R₁₀ représente un radical alkyle ayant de 1 à 12 atomes de carbone ou un radical alkényle ayant de 2 à 12 atomes de carbone on doit entendre des radicaux linéaires ou ramifiés éventuellement substitués par un ou plusieurs groupes hydroxyles ou un ou plusieurs atomes de fluor.

Par reste d'aminoacide on doit entendre un reste dérivant par exemple de l'un des 20 aminoacides de configuration L ou D (ou leur mélange racémique) constitutifs de protéines de mammifère.

Par reste d'un sucre on doit entendre un reste dérivant par exemple de glucose, galactose ou mannose.

Par reste d'un sucre aminé, on doit entendre un reste dérivant par exemple de glucosamine, de galactosamine ou de mannosamine.

Par hétérocycle, on entend de préférence un radical pipéridino, morpholino, pyrrolidino ou pipérazino, éventuellement substitué en position 4 par un radical alkyle en C₁-C₆ ou mono ou polyhydroxyalkyle tels que définis ci-dessus.

Parmi les composés de formule (I) ci-dessus, on peut notamment citer les suivants :
Acide 4-(5,6,7,8-tétrahydro-5,5,8,8-tétraméthyl-2-naphtylglyoxyloyloxy)benzoïque,
Acide 4-(5,6,7,8-tétrahydro-5,5,8,8-tétraméthyl-2-naphtylacétoxy) benzoïque,
Acide 4-(5,6,7,8-tétrahydro-5,5,8,8-tétraméthyl-2-naphtylacétamido) benzoïque,
Acide 4-(α-méthylène-5,6,7,8-tétrahydro-5,5,8,8-tétraméthyl-2-naphtylacétoxy)benzoïque,
Acide 4-(5,6,7,8-tétrahydro-5,5,8,8-tétraméthyl-2-naphtoyloxyméthyl)benzoïque,
Acide 4-(5,6,7,8-tétrahydro-5,5,8,8-tétraméthyl-2-naphtoylthiométhyl)benzoïque,
Acide 4-(5,6,7,8-tétrahydro-5,5,8,8-tétraméthyl-2-naphtyl carboxamidométhyl)benzoïque,
Acide 4-(N-méthyl-5,6,7,8-tétrahydro-5,5,8,8-tétraméthyl-2-naphtylcarboxamidométhyl)benzoïque,
Acide 4-(5,6,7,8-tétrahydro-5,5,8,8-tétraméthyl-2-naphtylméthyloxy carbonyl)benzoïque,
Acide 4-[5,6,7,8-tétrahydro-5,5,8,8-tétraméthyl-2-naphthyl (méthylthio)carbonyl]benzoïque,
Acide 5-(5,6,7,8-tétrahydro-5,5,8,8-tétraméthyl-2-naphtoyloxy méthyl)-2-thiophènecarboxylique,
Acide 4-(5,6,7,8,-tétrahydro-5,5,8,8-tétraméthyl-2-naphtyloxy carboxamido)benzoïque
Acide 4-(5,6,7,8-tétrahydro-5,5,8,8-tétraméthyl-2-naphtyl carbonyldioxy)benzoïque,
Acide 4-[1-(5,6,7,8-tétrahydo-5,5,8,8-tétraméthyl-2-naphtoyloxy) éthyl]benzoïque,
Acide 4-[[1-(5,6,7,8-tétrahydro-5,5,8,8-tétraméthyl-2-naphtyl) éthyloxy]carbonyl]benzoïque,
Acide 4-(3,5-di-tert-butyl-4-hydroxybenzoyloxyméthyl)benzoïque,
Acide 4-[3-(1-adamantyl)-4-méthoxybenzoyloxyméthyl]benzoïque,
Acide 4-(3-tert-butyl-4-méthoxybenzoyloxyméthyl)benzoïque,
Acide 4-(4-tert-butylbenzoyloxyméthyl)benzoïque,
Acide 4-[4-(1-adamantyl)-3-méthoxybenzoyloxyméthyl)]benzoïque,
Acide 4-[3-(1-adamantyl)-4-méthoxyphénoxycarboxamido]benzoïque,
Acide 4-[3-(1-adamantyl)-4-méthoxyphénylcarbonyldioxy]benzoïque,
Acide 4-[3-(1-adamantyl)-4-méthoxyphénylacétamido]benzoïque,
Acide 4-(5,6,7,8-tétrahydro-5,5,8,8-tétraméthyl-2-naphtoyl formamido)benzoïque,
Acide 4-(α-hydroxy-5,6,7,8-tétrahydro-5,5,8,8-tétraméthyl-2-naphtyl acétamido)benzoïque,
Acide 4-(5,6,7,8-tétrahydro-5,5,8,8-tétraméthyl-2-naphtylcarbamoylméthyl)benzoïque,
Acide 2-hydroxy-4-(5,6,7,8-tétrahydro-5,5,8,8 tétraméthyl-2-naphtylacétoxy) benzoïque,
Acide 4-(5,6,7,8-tétrahydro-5,5,8,8-tétraméthyl-2-naphtyloxycarbonylméthyl)benzoïque,
Acide 4-(N-méthyl-5,6,7,8-tétrahydro-5,5,8,8-tétraméthyl-2-naphtylacétamido)benzoïque,
Acide 4-(α-fluoro-5,6,7,8-tétrahydro-5,5,8,8-tétraméthyl-2-naphtylacétamido)benzoïque,
Acide 4-[3-(1-adamantyl)-4-méthoxyphényluréido]benzoïque,
Acide 2-hydroxy-4-(5,6,7,8-tétrahydro-5,5,8,8-tétraméthyl -2-naphtoylméthyloxy)benzoïque,
Acide 4-[2-(5,6,7,8-tétrahydro-5,5,8,8-tétraméthyl-2-naphtyl)propionamido]benzoïque
Divers schémas réactionnels peuvent être envisagés pour l'obtention des composés de formule (I), dans le cas où Y est un atome d'oxygène.

On peut citer les schémas réactionnels suivants :
Dans le cas où X est soit (i) soit (iv) 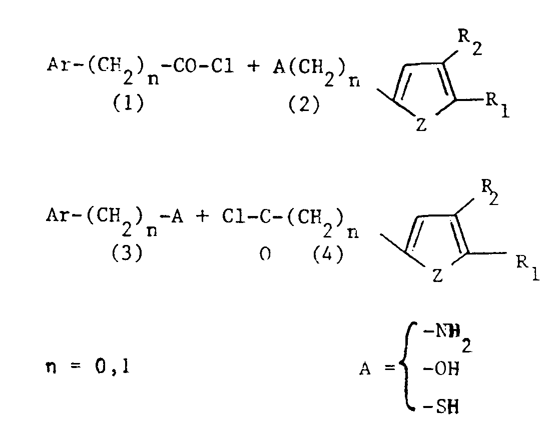
L'étape principale de cette préparation consiste à faire réagir en milieu anhydre, dans un solvant organique tel que le tétrahydrofuranne ou le chlorure de méthylène contenant une amine tertiaire (triéthylamine) ou de la pyridine ou un hydrure alcalin (hydrure de sodium), une forme activée d'un acide aromatique substitué ou d'un acide arylacétique substitué par exemple un chlorure d'acide (1) ou (4) ou un anhydride mixte, sur un composé aromatique porteur d'une fonction hydroxy ou amino ou thiol (2) ou (3), la réaction étant conduite à température ambiante et sous agitation.

Lorsque R₁ représente le radical -COOH, les composés sont préparés en protégeant R₁ par un groupe protecteur de type allylique, benzylique ou tert-butylique.

Le passage à la forme libre peut être effectué :
- dans le cas d'un groupe protecteur allylique, au moyen d'un catalyseur tel que certains complexes de métaux de transition en présence d'une amine secondaire,
- dans le cas d'un groupe protecteur benzylique, par débenzylation en présence d'hydrogène, au moyen d'un catalyseur tel que le palladium sur charbon,
- dans le cas d'un groupe protecteur tert-butylique au moyen d'iodure de triméthylsilyle.

Dans le cas où X est soit (ii) soit (v) 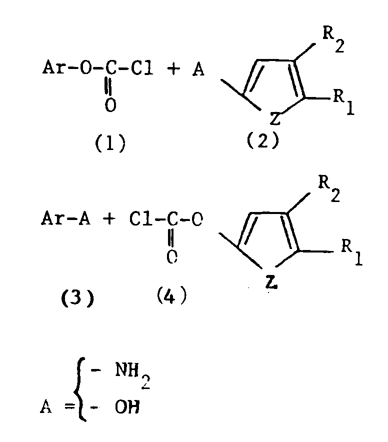
L'étape principale de cette préparation consiste à faire réagir en milieu anhydre dans un solvant organique tel que le chlorure de méthylène contenant une amine tertiaire (triéthylamine) ou de la pyridine un chloroformiate (1) ou (4) préparé par exemple à partir d'un dérivé hydroxyaryle et du chloroformiate de trichlorométhyle ou du phosgène, avec un composé aromatique porteur d'une fonction hydroxy ou amino (2) ou (3). La réaction étant conduite à température ambiante et sous agitation.

Dans le cas où R₁ représente le radical -COOH, les composés sont préparés de préférence en protégeant R₁ par un groupe protecteur benzylique. Le passage à la forme libre s'effectue alors par débenzylation en présence d'hydrogène, au moyen d'un catalyseur tel que le palladium sur charbon.

Dans le cas où X est (iii) et si Z n'est pas un atome d'oxygène, 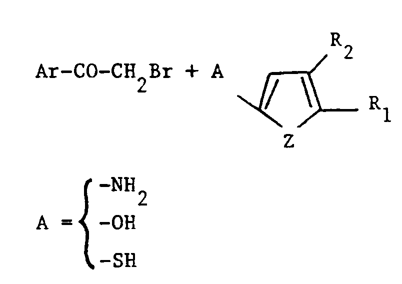
L'étape principale de cette préparation consiste à faire réagir en présence de carbonate de potassium ou d'un hydrure alcalin (hydrure de sodium) ou par transfert de phase en utilisant par exemple le bromure de tétrabutylammonium comme sel d'ammonium quaternaire, une alpha-bromocétone aromatique avec un composé aromatique porteur d'une fonction hydroxy ou amino ou thiol en para du radical R₁.

Dans le cas où R₁ représente le radical -COOH, les composés sont préparés de préférence en protégeant R₁ par un groupe protecteur allylique. Le passage à la forme libre s'effectue au moyen d'un catalyseur tel que le tetrakis (triphénylphosphine) palladium (0) en présence d'une amine secondaire (morpholine).

Les acides ainsi obtenus peuvent être convertis de manière connue en chlorure d'acide correspondant qui, traité par un alcool (R₆OH) ou une amine HN(r')(r'') donne l'ester ou l'amide correspondant.

La présente invention a également pour objet à titre de médicament les composés de formule (I) telle que définie ci-dessus.

Les composés selon l'invention présentent une bonne stabilité à la lumière et à l'oxygène.

Ces composés présentent une bonne activité dans le test de différenciation des cellules (F9) de tératocarcinome embryonnaire de la souris (Cancer Research 43, p. 5268, 1983) et/ou dans le test d'inhibition de l'ornithine décarboxylase après induction par "tape stripping" chez le rat nu (Lab. Animals 21, p.233-240, 1987) ou par le TPA chez la souris (Cancer Research 38, p.793-801, 1978). Ces tests montrent les activités des composés respectivement dans les domaines de la différenciation et de la prolifération.

Les composés selon l'invention conviennent particulièrement bien dans les domaines de traitement suivants :
1) Pour traiter les affections dermatologiques liées à un désordre de la kératinisation portant sur la différenciation et sur la prolifération notamment pour traiter les acnés vulgaires, comédoniennes, polymorphes, les acnés nodulokystiques, conglobata, les acnés séniles, les acnés secondaires telles que l'acné solaire, médicamenteuse, professionnelle.
2) Pour traiter d'autres types de trouble de la kératinisation, notamment les ichtyoses, les états ichtyosiformes, la maladie de Darier, les kératodermies palmoplantaires, les leucoplasies et les états leucoplasiformes, le lichen.
3) Pour traiter d'autres affections dermatologiques liées à un trouble de la kératinisation avec une composante inflammatoire et/ou immuno-allergique et, notamment, toutes les formes de psoriasis qu'il soit cutané, muqueux ou unguéal, et même le rhumatisme psoriatique, ou encore l'atopie cutanée, telle que l'eczéma, ou l'atopie respiratoire ; les composés peuvent également être utilisés dans certaines affections inflammatoires ne présentant pas de trouble de la kératinisation.
4) Pour traiter toutes les proliférations dermiques ou épidermiques qu'elles soient bénignes ou malignes, qu'elles soient d'origine virale telles que verrues vulgaires, les verrues planes et l'épidermodysplasie verruciforme, les proliférations pouvant également être induites par les ultra-violets notamment dans le cas des épithélioma baso et spino cellulaires.
5) Pour traiter d'autres désordres dermatologiques tel s que les dermatoses bulleuses et les maladies du collagène.
6) Pour traiter certains troubles ophtalmologiques, notamment les cornéopathies.
7) Pour réparer ou lutter contre le vieillissement de la peau, qu'il soit photoinduit ou chronologique.
8) Pour prévenir ou guérir les stigmates de l'atrophie épidermique et/ou dermique induite par les corticostéroïdes locaux ou systémiques, ou tout autre forme d'atrophie cutanée.
9) Pour favoriser la cicatrisation.
10) Pour lutter contre les troubles de la fonction sébacée tels que la séborrhée de l'acné ou la séborrhée simple.

La présente invention a également pour objet des compositions médicamenteuses contenant au moins un composé de formule (I) telle que définie ci-dessus, ou un de ses sels.

La présente invention a donc aussi pour objet une nouvelle composition médicamenteuse destinée notamment au traitement des affections susmentionnées, caractérisée par le fait qu'elle comporte, dans un support pharmaceutiquement acceptable, au moins un composé de formule (I) et/ou un de ses sels.

Les composés selon l'invention sont généralement administrés à une dose journalière d'environ 0,01 mg/kg à 100 mg/kg en poids corporel.

L'administration peut être effectuée par voie entérale, parentérale, topique ou oculaire. Par voie entérale, les médicaments peuvent se présenter sous forme de comprimés, de gelules, de dragées, de sirops, de suspensions, de solutions, de poudres, de granulés, d'émulsions. Par voie parentérale, les compositions peuvent se présenter sous forme de solutions ou suspensions pour perfusion ou pour injection.

Par voie topique, les compositions pharmaceutiques à base de composés selon l'invention sont destinées au traitement de la peau et des muqueuses et se présentent sous forme d'onguents, de crèmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques ou d'hydrogels permettant une libération contrôlée.

Ces compositions par voie topique peuvent se présenter soit sous forme anhydre, soit sous forme aqueuse selon l'indication clinique.

Par voie oculaire, ce sort principalement des collyres.

Ces compositions contiennent au moins un composé de formule (I) telle que définie ci-dessus ou un des ses sels, à une concentration de préférence comprise entre 0,0001 et 5 % par rapport au poids total de la composition.

Les composés de formule (I), selon l'invention, trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les effets néfastes du soleil ou dans le traitement des peaux physiologiquement sèches.

La présente invention vise donc également une composition cosmétique contenant, dans un support cosmétiquement acceptable, au moins un composé de formule (I) ou un de ses sels, cette composition se présentant notamment sous forme d'une crème, d'un lait, d'une lotion, d'un gel, de microsphères ou nanosphères ou vésicules lipidiques ou polymériques, d'un savon ou d'un shampooing,

La concentration en composé de formule (I), dans les compositions cosmétiques est comprise entre 0,0001 et 0,1 % en poids et de préférence entre 0,001 et 0,01 % en poids.

Les compositions médicamenteuses et cosmétiques selon l'invention peuvent contenir des additifs inertes ou même pharmacodynamiquement ou cosmétiquement actifs et notamment : des agents hydratants comme la thiamorpholinone et ses dérivés ou l'urée ; des agents antiséborrhéiques ou antiacnéiques, tels que la S-carboxyméthylcystéine, la S-benzyl-cystéamine, leurs sels et leurs dérivés, la tioxolone ou le peroxyde de benzoyle ; des antibiotiques comme l'érythromycine et ses esters, la néomycine, la clindamycine et ses esters, les tétracyclines, les agents antifongiques tels que le kétoconazole ou les polyméthylène-4,5 isothiazolinones-3 ; des agents favorisant la repousse des cheveux, comme le "Minoxidil" (2,4-diamino-6-pipéridino-pyrimidine-3-oxyde) et ses dérivés, le Diazoxide (7-chloro 3-méthyl 1,2,4-benzothiadiazine 1,1-dioxyde) et le Phénytoïn (5,5-diphényl-imidazolidine 2,4-dione) ; des agents anti-inflammatoires stéreoïdiens et non stéroïdiens ; des caroténoïdes et, notamment le β-carotène ; des agents anti-psoriatiques tels que l'anthraline et ses dérivés et les acides eicosa-5,8,11,14-tétraynoïque et eicosa-5,8,11-triynoïque-, leurs esters et leurs amides.

Les compositions selon l'invention peuvent également contenir des agents d'amélioration de la saveur, des agents conservateurs tels que les esters de l'acide parahydroxybenzoïque, des agents stabilisants, des agents régulateurs d'humidité, des agents régulateurs de pH, des agents modificateurs de pression osmotique, des agents émulsionnants, des filtres UV-A et UV-B, des anti-oxydants tels que l'α-tocophérol, le butylhydroxyanisole ou le butylhydroxytoluène.

On va maintenant donner, à titre d'illustration et sans aucun caractère limitatif, plusieurs exemples de préparation des composés actifs de formule (I) selon l'invention ainsi que des exemples de compositions les contenant.

### EXEMPLES DE PREPARATION

### EXEMPLE 1

### Acide 4-(5,6,7,8-tétrahydro-5,5,8,8-tétraméthyl-2-naphtylglyoxyloyloxy) benzoïque

(a) 5,6,7,8-tétrahydro-5,5,8,8-tétraméthyl-2-naphtylglyoxylate d'éthyle.
   Dans un tricol, on introduit 14,5 g (0,109 mole) de chlorure d'aluminium, 100 ml de dichlorométhane et ajoute goutte à goutte un mélange de 11,7 g (62 mmoles) de 5,6,7,8-tétrahydro-5,5,8,8-tétraméthylnaphtalène et de 8 ml (71 mmoles) de chlorure d'éthoxallyle dans 100 ml de dichlorométhane et agite à température ambiante pendant une heure. On verse le milieu réactionnel dans l'eau, extrait avec de l'éther éthylique, décante la phase organique, sèche sur sulfate de magnésium et évapore. Le résidu obtenu est purifié par chromatographie sur colonne de silice, élué avec un mélange de dichlorométhane et d'hexane (30-70). Après évaporation des solvants, on recueille 16,5 g (93 %) de l'ester attendu sous forme d'une huile légèrement jaune.
(b) Acide 5,6,7,8-tétrahydro-5,5,8,8-tétraméthyl-2-naphtylglyoxylique.
   Dans un ballon, on introduit 16 g (55,6 mmoles) de l'ester obtenu ci-dessus en (a) et 50 ml d'alcool éthylique. On ajoute une solution de 2,5 g (58,5 mmoles) d'hydroxyde de sodium dans 50 ml d'eau et chauffe à reflux pendant une heure. On évapore à sec le milieu réactionnel, reprend par l'eau, extrait avec de l'éther éthylique, décante la phase aqueuse, acidifie à pH 1 avec de l'acide chlorhydrique concentré, extrait avec de l'éther éthylique, décante la phase organique, sèche sur sulfate de magnésium et évapore. On recueille 14 g (97 %) d'acide 5,6,7,8-tétrahydro-5,5,8,8-tétraméthyl-2-naphtylglyoxylique, sous forme d'une huile incolore.
(c) Chlorure de 5,6,7,8-tétrahydro-5,5,8,8-tétraméthyl-2-naphtylglyoxyloyle.
   Dans un ballon, on introduit 2,6 g (10 mmoles) d'acide 5,6,7,8,-tétrahydro-5,5,8,8-tétraméthyl-2-naphtylglyoxylique dans 50 ml de dichlorométhane, ajoute 2 ml (10 mmoles) de dicyclohexylamine et agite à température ambiante pendant une heure. On ajoute ensuite goutte à goutte 800 µl (10 mmoles) de chlorure de thionyle et agite à température ambiante pendant une heure. On évapore à sec, reprend par 200 ml d'éther éthylique, filtre le sel de dicyclohexylamine et évapore. On obtient 2,8 g (100 %) de chlorure d'acide brut, qui est utilisé tel quel pour la suite de la synthèse.
(d) 4-(5,6,7,8-tétrahydro-5,5,8,8-tétraméthyl-2-naphtylglyoxyloyloxy)benzoate de tert-butyle.
   Dans un ballon, on introduit 1,94 g (10 mmoles) de 4-hydroxybenzoate de tert-butyle, 1,4 ml (10 mmoles) de triéthylamine et 50 ml de THF. On ajoute goutte à goutte une solution de 2,8 g (10 mmoles) du chlorure d'acide obtenu ci-dessus en (c) dans 50 ml de THF et agite à température ambiante pendant quatre heures. On verse le milieu réactionnel dans l'eau, extrait avec de l'éther éthylique, décante la phase organique, sèche sur sulfate de magnésium et évapore. Le résidu obtenu est purifié par chromatographie sur colonne de silice, élué avec un mélange de dichlorométhane et d'hexane (40-60). Après évaporation des solvants, on recueille 2 g (46 %) d'ester tert-butylique sous forme d'une huile incolore.
(e) Acide 4-(5,6,7,8-tétrahydro-5,5,8,8-tétraméthyl-2-naphtylglyoxyloyloxyl)benzoïque.
   Dans un ballon, on introduit 1,6 g (3,6 mmoles) d'ester obtenu ci-dessus en (d) et 50 ml de tétrachlorure de carbone. On refroidit avec un bain de glace, ajoute goutte à goutte 520 µl (3,6 mmoles) d'iodure de triméthylsilane et agite à température ambiante pendant quatre heures. On verse le milieu réactionnel dans l'eau, extrait avec de l'éther éthylique, décante la phase organique, sèche sur sulfate de magnésium et évapore. Le résidu obtenu est purifié par chromatographie sur colonne de silice, élué avec un mélange de dichlorométhane et d'hexane (95-5). On recueille après évaporation des solvants, 830 mg (59 %) d'acide 4-(5,6,7,8-tétrahydro-5,5,8,8-tétraméthyl-2-naphtylglyoxyloyloxyl)benzoïque de point de fusion : 160-161°C.

### EXEMPLE 2

### Acide 4-(5,6,7,8-tétrahydro-5,5,8,8-tétraméthyl-2-naphtylacétoxy) benzoïque.

(a) Acide 5,6,7,8-tétrahydro-5,5,8,8-tétraméthyl-2-naphtylacétique.
   Dans un ballon, on introduit 4,68 g (18 mmoles) d'acide 5,6,7,8-tétrahydro-5,5,8,8-tétraméthyl-2-naphtylglyoxylique, 50 ml d'hydrate d'hydrazine et chauffe à reflux jusqu'à dissolution. A 100°C, on ajoute par petites quantités 8 g d'hydroxyde de potassium et chauffe à reflux pendant quatre heures. On évapore à sec le milieu réactionnel, reprend par l'eau, acidifie à pH 1 avec de l'acide chlorhydrique concentré, extrait avec de l'éther éthylique, décante la phase organique, puis sèche sur sulfate de magnésium et évapore. Le résidu obtenu est purifié par chromatographie sur colonne de silice, élué avec un mélange de dichlorométhane et d'éther éthylique (95-5). Après évaporation des solvants, on recueille 3,8 g (86 %) d'acide 5,6,7,8-tétrahydro-5,5,8,8-tétraméthyl-2-naphtylacétique, de point de fusion : 149-150°C.
(b) Chlorure de 5,6,7,8-tétrahydro-5,5,8,8-tétraméthyl-2-naphtylacétyle.
   6,6 g (26,8 mmoles) d'acide 5,6,7,8-tétrahydro-5,5,8,8-tétraméthyl-2-naphtylacétique et 20 ml de chlorure de thionyle sont chauffés à reflux jusqu'à cessation de dégagement gazeux. On évapore à sec et obtient 7,1 g (100 %) du chlorure d'acide brut, qui est utilisé tel quel pour la suite de la synthèse.
(c) 4-(5,6,7,8-tétrahydro-5,5,8,8-tétraméthyl-2-naphtylacétoxy) benzoate de benzyle.
   De manière analogue à l'exemple 1(d) par réaction de 1,93 g (7,7 mmoles) de chlorure d'acide obtenu ci-dessus en 2(b) avec 1,75 g (7,7 mmoles) de 4-hydroxybenzoate de benzyle, on obtient 2,3 g (65 %) d'ester benzylique, sous forme d'une huile translucide.
(d) Acide 4-(5,6,7,8-tétrahydro-5,5,8,8-tétraméthyl-2-naphtylacétoxy)benzoïque.
   Dans un réacteur, on introduit 2,4 g (5,2 mmoles) de 4-(5,6,7,8-tétrahydro-5,5,8,8-tétraméthyl-2-naphtylacétoxy)benzoate de benzyle, 1 g de palladium sur charbon (5 %) et 100 ml de dioxanne. On hydrogène à température ambiante et sous une pression de 7 bars pendant trois heures, filtre le catalyseur, lave avec deux fois 50 ml de THF, et évapore les filtrats. Le résidu obtenu est trituré dans le minimum d'éther éthylique, filtré et séché. On obtient 1,35 g (70 %) d'acide 4-(5,6,7,8-tétrahydro-5,5,8,8-tétraméthyl-2-naphtylacétoxy)benzoïque de point de fusion : 142-143°C.

### EXEMPLE 3

### Acide 4-(5,6,7,8-tétrahydro-5,5,8,8-tétraméthyl-2-naphtylacétamido) benzoïque.

(a) 4-aminobenzoate d'allyle.
   Dans un ballon, on introduit 50 ml d'alcool allylique, ajoute par petites quantités 750 mg (22 mmoles) de sodium et agite jusqu'à réaction totale du métal. On introduit ensuite 30,2 g (0,2 mole) de 4-aminobenzoate d'éthyle dissous dans 200 ml d'alcool allylique, chauffe à reflux et distille jusqu'à obtention de 180 ml de distillat. On évapore le milieu réactionnel, reprend par l'eau et l'éther éthylique, décante la phase organique, sèche sur sulfate de magnésium et évapore. Le résidu obtenu est trituré dans 200 ml d'hexane et filtré. On recueille 29 g (82 %) d'ester allylique, de point de fusion : 48-50°C.
(b) 4-(5,6,7,8-tétrahydro-5,5,8,8-tétraméthyl-2-naphtylacétamido) benzoate d'allyle.
   De manière analogue à l'exemple 1(d) par réaction de 4,5 g (16,8 mmoles) de chlorure de 5,6,7,8-tétrahydro-5,5,8,8-tétraméthyl-2-naphtylacétyle avec 3 g (16,8 mmoles) de 4-aminobenzoate d'allyle, on obtient 5,1 g (75 %) de l'ester attendu, sous forme d'une huile légèrement jaune.
(c) Acide 4-(5,6,7,8-tétrahydro-5,5,8,8-tétraméthyl-2-naphtylacétamido)benzoïque.
   Dans un tricol et sous courant d'azote, on introduit 2,15 g (5,3 mmoles) de l'ester obtenu ci-dessus en (b), 346 mg (0,3 mmole) de tétrakis(triphénylphosphine)palladium (O) et 50 ml de THF anhydre, on ajoute goutte à goutte 4,6 ml (53 mmoles) de morpholine et agite à température ambiante pendant quatre heures. On évapore à sec le milieu réactionnel, triture dans l'éther éthylique, puis filtre le sel de morpholine formé. On introduit le sel dans 100 ml d'eau, acidifie à pH 1 avec de l'acide chlorhydrique concentré, extrait avec de l'éther éthylique, décante la phase organique, puis sèche sur sulfate de magnésium et évapore. Le résidu obtenu est purifié par chromatographie sur colonne de silice élué avec un mélange de dichlorométhane et d'éther éthylique (95-5). Après évaporation des solvants, on recueille 1,5 g (79 %) d'acide 4-(5,6,7,8-tétrahydro-5,5,8,8-tétraméthyl-2-naphtylacétamido)benzoïque, de point de fusion : 203-204°C.

### EXEMPLE 4

### Acide 4-(α-méthylène-5,6,7,8-tétrahydro-5,5,8,8-tétraméthyl-2-naphtylacétoxy)benzoïque.

(a) Acide α-hydroxy-α-méthyl-5,6,7,8-tétrahydro-5,5,8,8-tétraméthyl-2-naphtylacétique.
   Dans un tricol, on introduit 3,1 g (12 mmoles) d'acide 5,6,7,8-tétrahydro-5,5,8,8-tétraméthyl-2-naphtylglyoxylique et 50 ml de THF. A 0°C et sous courant d'azote, on ajoute goutte à goutte 16 ml (48 mmoles) d'une solution d'iodure d'éthyle magnésium dans l'éther éthylique (3M) et agite à température ambiante pendant quatre heures. On verse le milieu réactionnel dans l'eau, extrait avec de l'éther éthylique, décante la phase organique, puis sèche sur sulfate de magnésium et évapore. Le résidu obtenu est purifié par chromatographie sur colonne de silice, élué avec un mélange de dichlorométhane et d'éther éthylique (20-80). Après évaporation des solvants, on recueille 2,6 g (79 %) du produit attendu, de point de fusion : 168-169°C.
(b) Acide α-méthylène-5,6,7,8-tétrahydro-5,5,8,8-tétraméthyl-2-naphtylacétique.
   A 2,4 g (8,7 mmoles) de l'acide obtenu ci-dessus en (a) dans 80 ml de dioxanne, on ajoute 4,4 ml d'acide chlorhydrique concentré et chauffe à reflux pendant quatre heures. On évapore à sec le milieu réactionnel, reprend par l'eau et l'éther éthylique, décante la phase organique, puis sèche sur sulfate de magnésium et évapore. Le résidu obtenu est purifié par chromatographie sur colonne de silice en éluant avec de l'éther éthylique. On recueille 2 g (91 %) de l'acide attendu, de point de fusion : 149-150°C.
(c) α-méthylène-5,6,7,8-tétrahydro-5,5,8,8-tétraméthyl-2-naphtylacétoxybenzoate de tert-butyle.
   Dans un ballon, on introduit 1,9 g (7,3 mmoles) d'acide obtenu ci-dessus en (b), 1,5 g (7,3 mmoles) de 4-hydroxybenzoate de tert-butyle et 100 ml de THF. On ajoute successivement 1,5 g (7,3 mmoles) de N,N'-dicyclohexyl-carbodiimide, 0,9 g (7,3 mmoles) de 4-diméthylaminopyridine et agite à température ambiante pendant quatre heures. On verse le milieu réactionnel dans l'eau, extrait avec de l'éther éthylique, décante la phase organique, sèche sur sulfate de magnésium et évapore. Le résidu obtenu est purifié par chromatographie sur colonne de silice en éluant avec du dichlorométhane. On recueille 2,3 g (73 %) de l'ester attendu, sous forme d'une huile incolore.
(d) Acide 4-(α méthylène-5,6,7,8-tétrahydro-5,5,8,8-tétraméthyl-2-naphtylacétoxy)benzoïque.
   De manière analogue à l'exemple 1(e) à partir de 2,1 g (4,8 mmoles) de l'ester obtenu ci-dessus en (c), on obtient 1,7 g (95 %) d'acide attendu de point de fusion : 186-187°C.

### EXEMPLE 5

### Acide 4-(5,6,7,8-tétrahydro-5,5,8,8-tétraméthyl-2-naphtcyloxyméthyl) benzoïque.

(a) 4-formylbenzoate de benzyle.
   Dans un ballon et sous courant d'azote, on introduit 1,8 g (60 mmoles) d'hydrure de sodium (80 % dans l'huile) et 50 ml de DMF. On ajoute goutte à goutte 7,5 g (50 mmoles) d'acide 4-formylbenzoïque dissous dans 100 ml DMF et agite à température ambiante jusqu'à cessation du dégagement gazeux. On introduit ensuite goutte à goutte une solution de 7,1 ml (60 mmoles) de bromure de benzyle dans 50 ml de DMF et agite à température ambiante pendant quatre heures. On verse le milieu réactionnel dans l'eau, extrait avec de l'éther éthylique, décante la phase organique, sèche sur sulfate de magnésium et évapore. Le résidu obtenu est purifié par chromatographie sur colonne de silice, élué avec un mélange de dichlorométhane et d'hexane (50-50). On recueille, après évaporation des solvants, 10,9 g (91 %) d'ester benzylique attendu sous forme d'une huile incolore.
(b) Hydroxyméthylbenzoate de benzyle.
   Dans un ballon, on introduit 10,3 g (43 mmoles) de 4-formylbenzoate de benzyle, 50 ml de THF et 50 ml d'alcool méthylique. Tout en refroidissant avec un bain de glace, on ajoute par petites quantités 820 mg (21,5 mmoles) de borohydrure de sodium et agite à température ambiante pendant deux heures. On verse le milieu réactionnel dans l'eau, extrait avec de l'éther éthylique, décante la phase organique, sèche sur sulfate de magnésium et évapore. On recueille 10,4 g (100 %) du produit attendu de point de fusion : 55-56°C.
(c) 4-(5,6,7,8-tétrahydro-5,5,8,8-tétraméthyl-2-naphtoyloxyméthyl)benzoate de benzyle.
   De manière analogue à l'exemple 1(d), par réaction de 2,78 g (10 mmoles) de chlorure de l'acide 5,6,7,8-tétrahydro-5,5,8,8-tétraméthyl-2-naphtoyle avec 2,42 g (10 mmoles) de 4-hydroxybenzoate de benzyle, on obtient 3,7 g (81 %) d'ester benzylique, sous forme d'une huile incolore.
(d) Acide 4-(5,6,7,8-tétrahydro-5,5,8,8-tétraméthyl-2-naphtoyloxyméthyl)benzoïque.
   De manière analogue à l'exemple 2(d), à partir de 2,2 g (4,8 mmoles) de l'ester obtenu ci-dessus en (c), on obtient 800 mg (46 %) d'acide 4-(5,6,7,8-tétrahydro-5,5,8,8-tétraméthyl-2-naphtoyloxyméthyl)benzoïque de point de fusion : 176-177°C.

### EXEMPLE 6

### Acide 4-(3,5-di-tert-butyl-4-hydroxybenzoyloxyméthyl)benzoïque.

(a) 4-(3,5-di-tert-butyl-4-hydroxybenzoyloxyméthyl)benzoate de benzyle.
   De manière analogue à l'exemple 1(d), par réaction de 2,33 g (10 mmoles) de chlorure de 3,5-di-tert-butyl-4-hydroxybenzoyle avec 2,42 g (10 mmoles) de 4-hydroxybenzoate de benzyle, on obtient 2,9 g (61 %) d'ester benzylique, de point de fusion : 110-111°C.
(b) Acide 4-(3,5-di-tert-butyl-4-hydroxybenzoyloxyméthyl)benzoïque
   De manière analogue à l'exemple 2(d) à partir de 1,4 g (3,1 mmoles) de l'ester obtenu ci-dessus en (a), on obtient 535 mg (45 %) d'acide 4-(3,5-di-tert-butyl-4-hydroxybenzoyloxyméthyl)benzoïque, de point de fusion : 171-172°C.

### EXEMPLE 7

### Acide 4-[3-(1-adamantyl)-4-méthoxybenzoyloxyéthyl]benzoïque.

(a) 4-formylbenzoate d'allyle.
   Dans un ballon et sous courant d'azote, on introduit 3,3 g (0,11 mole) d'hydrure de sodium (80 % dans l'huile) et 100 ml de DMF. On ajoute goutte à goutte 15 g (0,1 mole) d'acide 4-formylbenzoïque dissous dans 200 ml de DMF et agite à température ambiante jusqu'à cessation de dégagement gazeux. On introduit ensuite 9,5 ml (0,11 mole) de bromure d'allyle et agite à température ambiante pendant quatre heures. On verse le milieu réactionnel dans l'eau, extrait avec de l'éther éthylique, décante la phase organique, puis sèche sur sulfate de magnésium et évapore. On recueille 19g (100 %) d'ester allylique, sous forme d'une huile légèrement jaune.
(b) 4-hydroxyméthylbenzoate d'allyle.
   De manière analogue à l'exemple 5(b) à partir de 17,3 g (91 mmoles) du 4-formylbenzoate d'allyle, on obtient 15,1 g (86 %) du produit attendu, sous forme d'une huile incolore.
(c) 4-[3-(1-adamantyl)-4-méthoxybenzoyloxyméthyl]benzoate d'allyle.
   De manière analogue à l'exemple 1(d) par réaction de 3,1 g(10 mmoles) de chlorure de 3-(1-adamantyl)-4-méthoxybenzoyle avec 1,92 g (10 mmoles) de 4-hydroxybenzoate d'allyle, on obtient 2,9 g (63 %) d'ester allylique, sous forme d'une huile incolore.
(d) Acide 4-[3-(1-adamantyl)-4-méthoxybenzoyloxyméthyl]benzoïque.
   De manière analogue à l'exemple 3(c) à partir de 2,8 g (6,1 mmoles) de l'ester obtenu ci-dessus en (c), on obtient 1,6 g (62 %) d'acide 4- 3-(1-adamantyl)-4-méthoxybenzoyloxyméthyl benzoïque de point de fusion : 215-216°C.

### EXEMPLE 8

### Acide 4-(3-tert-butyl-4-méthoxybenzoyloxyméthyl)benzoïque.

(a) 4-(3-tert-butyl-4-méthoxybenzoyloxyméthyl)benzoate d'allyle
   De manière analogue à l'exemple 1(d), par réaction de 2,4 g (10 mmoles) de chlorure de 3-tert-butyl-4-méthoxybenzoyle avec 1,9 g (10 mmoles) de 4-hydroxyméthylbenzoate d'allyle, on obtient 2,65 g (69 %) d'ester allylique, sous forme d'une buile légèrement jaune.
(b) Acide 4-(3-tert-butyl-4-méthoxybenzoyloxyméthyl)benzoïque.
   De manière analogue l'exemple 3(c) à partir de 2,65 g (7 mmoles) de l'ester obtenu ci-dessus en (a), on obtient 1,55 g (64 %) d'acide 4-(3-tert-butyl-4-méthoxybenzoyloxyméthyl)benzoïque, de point de fusion : 171-172°C.

### EXEMPLE 9

### Acide 4-(4-tert-butylbenzoyloxyméthyl)benzoïque.

(a) 4-(4-tert-butylbenzoyloxyméthyl)benzoate d'allyle.
   De manière analogue à l'exemple 1(d), par réaction de 2 g (15 mmoles) de chlorure de 4-tert-butylbenzoyle avec 2,9 g (15 mmoles) de 4-hydroxyméthylbenzoate d'allyle, on obtient 4 g (77 %) d'ester allylique, sous forme d'une huile incolore.
(b) Acide 4-(4-tert-butylbenzoyloxyméthyl)benzoïque
   De manière analogue à l'exemple 3(c) à partir de 3,8 g (10,8 mmoles) de l'ester obtenu ci-dessus en (a), on obtient 3 g (89 %) d' acide 4-(4-tert-butylbenzoyloxyméthyl)benzoïque, de point de fusion : 153-154°C.

### EXEMPLE 10

### Acide 4-[4-(1-adamantyl)-3-méthoxybenzoyloxyméthyl]benzoïque.

(a) 2-(1-adamantyl)-5-bromophénol.
   Dans un ballon, on introduit 17 g (0,1 mole) de 3-bromophénol, 15,22 g (0,1 mole) de 1-adamantanol et 50 ml de dichlorométhane. On ajoute goutte à goutte 5 ml d'acide sulfurique concentré, et agite à température ambiante pendant 24 heures. On verse le milieu réactionnel dans l'eau, neutralise avec du bicarbonate de sodium, extrait avec de l'éther éthylique, décante la phase organique, puis sèche sur sulfate de magnésium et évapore. Le résidu obtenu est purifié par chromatographie sur colonne de silice, élué avec une mélange de dichlorométhane et d'hexane (50-50). Après évaporation des solvants, on recueille 15,2 g (50 %) du phénol attendu, de point de fusion : 112-114°C.
(b) 2-(1-adamantyl)-5-bromoanisole.
   Dans un ballon, on introduit 1,6 g (53 mmoles) d'hydrure de sodium (80 % dans l'huile), 50 ml de THF et ajoute goutte à goutte une solution de 15 g (49 mmoles) du phénol obtenu ci-dessus en (a) dissous dans 100 ml de DMF. On agite jusqu'à cessation du dégagement gazeux et introduit ensuite 3,1 ml (49 mmoles) d'iodure de méthyle. On agite à température ambiante quatre heures, verse le milieu réactionnel dans l'eau, décante la phase organique, puis sèche sur sulfate de magnésium et évapore. Le résidu obtenu est trituré dans le minimum d'hexane et filtré. On recueille 12,6 g (80 %) de 2-(1-adamantyl)-5-bromoanisole, de point de fusion : 159-160°C.
(c) Acide 4-(1-adamantyl)-3-méthoxybenzoïque.
   11,3 g, (35 mmoles) du composé obtenu ci-dessus en (b) est dissous dans 50 ml de THF anhydre. La solution obtenue est ajoutée goutte à goutte sur du magnésium (1,3 g, 53 mmoles) et un cristal d'iode. Après l'introduction des 5 premiers millilitres, on chauffe à reflux et maintient celui-ci deux heures après la fin de l'addition. On refroidit à -40°C et fait passer un courant de CO₂ pendant 30' puis laisse remonter la température à 20°C. On verse le milieu réactionnel dans l'eau, extrait avec de l'éther éthylique, décante la phase organique puis sèche sur sulfate de magnésium et évapore. Le résidu obtenu est purifié par chromatographie sur colonne de silice, élué avec un mélange de dichlorométhane et d'éther éthylique (90-10). Après évaporation des solvants, on recueille 5,1 g (51 %) d'acide 4-(1-adamantyl)-3-méthoxybenzoïque, de point de fusion : 259-260°C.
(d) Chlorure de 4-(1-adamantyl)-3-méthoxybenzoyle.
   De manière analogue à l'exemple 2(b) à partir de 2,9 g (10 mmoles) d'acide 4-(1-adamantyl)-3-méthoxybenzoïque, on obtient 3,1 g (100 %) de chlorure d'acide brut, qui est utilisé tel quel pour la suite de la synthèse.
(e) 4-[4-(1-adamantyl)-3-méthoxybenzoyloxyméthyl]benzoate d'allyle.
   De manière analogue à l'exemple 1(d) par réaction de 3,1 g (10 mmoles) du chlorure d'acide obtenu ci-dessus en (d) avec 1,92 g (10 mmoles) de 4-hydroxyméthylbenzoate d'allyle, on obtient 2,9 g (63 %) d'ester allylique, sous forme d'une huile translucide.
(f) Acide 4-[4-(1-adamantyl)-3-méthoxybenzoyloxyméthyl]benzoïque.
   De manière analogue à l'exemple 3(c) à partir de 2.7 g (5,8 mmoles) de l'ester obtenu ci-dessus en (e), on obtient 1 g (40 %) d'acide 4-[4-(1-adamantyl)-3-méthoxybenzoyloxyméthyl]benzoïque, de point de fusion : 206-207°C.

### EXEMPLE 11

### Acide 4-(5,6,7,8-tétrahydro-5,5,8,8-tétraméthyl-2-naphtcylthiométhyl) benzoïque.

(a) 4-acétylthiométhylbenzoate d'allyle.
   Dans un ballon on introduit 25,7 g (98 mmoles) de triphénylphosphine, 100 ml de THF et ajoute, goutte à goutte, à 0°C 19,8 g (98 mmoles) d'azodicarboxylate de diisopropyle dissous dans 150 ml de THF. On agite une heure à 0°C puis ajoute un mélange de 9,4 g (49 mmoles) de 4-hydroxyméthylbenzoate d'allyle et 7 ml (98 mmoles) d'acide thioacétique. On agite à température ambiante quatre heures, verse le milieu réactionnel dans l'eau, extrait avec de l'éther éthylique, décante la phase organique, puis sèche sur sulfate de magnésium et évapore. Le résidu obtenu est purifié par chromatographie sur colonne de silice, élué avec un mélange de dichlorométhane et d'hexane (60-40). On obtient 12,2 g (100 %) du produit attendu, sous forme d'une huile.
(b) Acide 4-mercaptométhylbenzoïque.
   Dans un ballon, on introduit 12,25 g (49 mmoles) de l'ester obtenu ci-dessus en (a), 100 ml de soude méthanolique 2N et chauffe à reflux quatre heures. On évapore à sec le milieu réactionnel, reprend par l'eau, acidifie à pH 1 avec de l'acide chlorhydrique concentré, extrait avec de l'éther éthylique, puis sèche sur sulfate de magnésium et évapore. Le résidu obtenu est trituré dans le minimum d'éther éthylique puis filtré. On obtient 6,8 g (83 %) d'acide 4-mercaptométhylbenzoïque de point de fusion : 173-174°C.
(c) Acide 4-(5,6,7,8-tétrahydro-5,5,8,8-tétraméthyl-2-naphtoylthiométhyl)benzoïque.
   Dans un ballon, on introduit 1,68 g (10 mmoles) d'acide 4-mercaptométhylbenzoïque, 2,8 ml (20 mmoles) de triéthylamine et 50 ml de THF. On ajoute, goutte à goutte, une solution de 2,5 g (10 mmoles) de chlorure de 5,6,7,8-tétrahydro-5,5,8,8-tétraméthyl-2-naphtcyle dans 50 ml de THF et agite à température ambiante pendant quatre heures. On verse le milieu réactionnel dans l'eau, extrait avec de l'éther éthylique, décante la phase organique, puis sèche sur sulfate de magnésium et évapore. Le résidu obtenu est trituré dans 50 ml d'un mélange d'éther éthylique et d'hexane (50-50) puis filtré. On obtient 2,5 g (66 %) d'acide attendu de point de fusion : 150-151°C.

### EXEMPLE 12

### Acide 4-(5,6,7,8-tétrahydro-5,5,8,8-tétraméthyl-2-naphtylcarboxamidométhyl) benzoïque.

(a) 4-(5,6,7,8-tétrahydro-5,5,8,8-tétraméthyl-2-naphtylcarboxamidométhyl)benzoate de méthyle.
   De manière analogue à l'exemple 1(d) par réaction de 5 g (20 mmoles) de chlorure de 5,6,7,8-tétrahydro-5,5,8,8-tétraméthyl-2-naphtoyle avec 3,3 g (20 mmoles) de 4-aminométhylbenzoate de méthyle, on obtient 7,2 g (95 %) d'ester méthylique, de point de fusion : 162-163°C.
(b) Acide 4-(5,6,7,8-tétrahydro-5,5,8,8-tétraméthyl-2-naphtylcarboxamidométhyl)benzoïque.
   3,8 g (10 mmoles) de l'ester méthylique obtenu ci-dessus en (a) dans 200 ml de soude méthanolique 2N sont agités à température ambiante pendant quatre heures. On évapore à sec le milieu réactionnel, reprend par l'eau, acidifie à pH 1 avec de l'acide chlorhydrique concentré, extrait avec de l'éther éthylique, décante la phase organique, puis sèche sur sulfate de magnésium et évapore. Le résidu obtenu est trituré dans le minimum d'éther éthylique et filtré. On recueille 3,2 g (86 %) d'acide attendu de point de fusion : 275-276°C.

### EXEMPLE 13

### Acide 4-(N-méthyl-5,6,7,8-tétrahydro-5,5,8,8-tétraméthyl-2-naphtylcarboxamidométhyl)benzoïque.

(a) 4-(N-méthyl-5,6,7,8-tétrahydro-5,5,8,8-tétraméthyl-2-naphtylcarboxamidométhyl)benzoate de méthyle.
   Dans un ballon on introduit 1,6 ml (11 mmoles) de diisopropylamine et 50 ml de THF. A -78°C et sous courant d'azote, on ajoute goutte à goutte 6,9 ml (11 mmoles) de n-butyl lithium (1,6M) et agite 30 minutes. Cette solution est ajoutée goutte à goutte à -78°C une solution de 3,8 g (10 mmoles) de l'ester méthylique obtenu en 12(a) dans 50 ml de THF. On agite pendant 30 minutes, ajoute 750 µl (12 mmoles) d'iodure de méthyle et laisse remonter la température à 20°C. On verse le milieu réactionnel dans l'eau, extrait avec de l'éther éthylique, décante la phase organique, puis sèche sur sulfate de magnésium et évapore. Le résidu obtenu est purifié par chromatographie sur colonne de silice, élué avec un mélange de dichlorométhane et d'éther éthylique (98-2). Après évaporation des solvants, on obtient 2,8 g (72 %) d'ester méthylique, de point de fusion : 142-143°C.
(b) Acide 4-(N-méthyl-5,6,7,8-tétrahydro-5,5,8,8-tétraméthyl-2-naphtylcarboxamidométhyl)benzoïque.
   De manière analogue à l'exemple 12(b) à partir de 1,2 g (30,5 mmoles) de l'ester obtenu ci-dessus en (a), on obtient 1 g (86 %) d'acide attendu de point de fusion : 181-182°C.

### EXEMPLE 14

### Acide 4-(5,6,7,8-tétrahydro-5,5,8,8-tétraméthyl-2-naphtylméthyloxycarbonyl) benzoïque

(a) 5,6,7,8-tétrahydro-5,5,8,8-tétraméthyl-2-naphtylméthanol.
   Dans un ballon, on introduit 3,8 g (0,1 mole) d'hydrure double de lithium et d'aluminium et 100 ml de THF. Sous courant d'azote, on ajoute goutte à goutte une solution de 11,6 g (0,05 mole) d'acide 5,6,7,8-tétrahydro-5,5,8,8-tétraméthyl-2-naphtoïque dans 100 ml de THF, et chauffe à reflux pendant quatre heures. On hydrolyse l'excès d'hydrure avec 7,2 ml d'une solution à 10 % de tartrate double de sodium et de potassium, filtre le précipité et évapore la phase organique. On recueille 10,6 g (97 %) de l'alcool attendu de point de fusion : 74-75°C.
(b) Téréphtalate de diallyle.
   De manière analogue à l'exemple 3(a) à partir de 19,4 g (0,1 mole) de téréphtalate de diméthyle, on obtient 18,3 g (75 %) du diester attendu, sous forme d'une huile.
(c) Acide 4-(allyloxycarbonyl)benzoïque.
   Dans un ballon, on introduit 12,3 g (50 mmoles) du diester obtenu ci-dessus en (b), 100 ml de THF, 10,5 g (0,25 mole) d'hydroxyde de lithium et chauffe à reflux pendant deux heures. On verse le milieu réactionnel dans 200 ml d'acide chlorhydrique (5N), filtre le solide, le lave deux fois avec 100 ml d'eau et le dissout dans l'éther éthylique. On sèche la phase organique sur sulfate de magnésium, évapore, purifie le résidu obtenu par chromatographie sur colonne de silice, élué avec de l'éther éthylique. On recueille 9 g (88 %) d'acide 4-(allyloxycarbonyl)benzoïque de point de fusion : 154-155°C.
(d) 4-(5,6,7,8-tétrahydro-5,5,8,8-tétraméthyl-2-naphtylméthyloxycarbonyl)benzoate d'allyle.
   De manière analogue à l'exemple 4(c) par réaction de 2,06 g (10 mmoles) d'acide 4-(allyloxycarbonyl)benzoïque avec 2,18 g (10 mmoles) de 5,6,7,8-tétrahydro-5,5,8,8-tétraméthyl-2-naphtylméthanol, on obtient 2,8 g (70 %) d'ester allylique, sous forme d'une huile translucide.
(e) Acide 4-(5,6,7,8-tétrahydro-5,5,8,8-tétraméthyl-2-naphtylméthyloxycarbonyl)benzoïque.
   De manière analogue à l'exemple 3(c) à partir de 2,6 g (6,4 mmoles) de l'ester obtenu ci-dessus en (d), on obtient 1,7 g (74 %) d' acide 4-(5,6,7,8-tétrahydro-5,5,8,8-tétraméthyl-2-naphtylméthyloxycarbonyl) benzoïque de point de fusion : 171-172°C.

### EXEMPLE 15

### Acide 4-[5,6,7,8-tétrahydro-5,5,8,8-tétraméthyl-2-naphtyl (méthylthio)carbonyl]benzoïque

(a) S-thioacétate de (5,6,7,8-tétrahydro-5,5,8,8-tétraméthyl-2-naphtylméthyle).
   De manière analogue à l'exemple 11(a) à partir de 4,36 g (20 mmoles) de 5,6,7,8-tétrahydro-5,5,8,8-tétraméthyl-2-naphtylméthanol, on obtient 5,1 g (93 %) du produit attendu.
(b) 5,6,7,8-tétrahydro-5,5,8,8-tétraméthyl-2-naphtylméthanethiol.
   De manière analogue à l'exemple 11(b), à partir de 4,8 g (17,4 mmoles) du produit obtenu ci-dessus en (a), on obtient 3,7 g (93 %) du thiol attendu, sous forme d'une huile légèrement jaune.
(c) 4-[5,6,7,8-tétrahydro-5,5,8,8-tétraméthyl-2-naphtyl (méthylthio)carbonyl]benzoate d'allyle.
   De manière analogue à l'exemple 4(c), par réaction de 2 g (10 mmoles) d'acide 4-(allyloxycarbonyl)benzoïque avec 2,34 g (10 mmoles) de 5,6,7,8-tétrahydro-5,5,8,8-tétraméthyl-2-naphthylméthanethiol, on obtient 3,4 g (81 %) d'ester allylique attendu de point de fusion : 79-80°C.
(d) Acide 4-[5,6,7,8-tétrahydro-5,5,8,8-tétraméthyl-2-naphtyl (méthylthio)carbonyl]benzoïque.
   De manière analogue à l'exemple 3(c) à partir de 3,3 g (7,8 mmoles) de l'ester obtenu ci-dessus en (c) , on obtient 2,7 g (90 %) d'acide 4-(5,6,7,8-tétrahydro-5,5,8,8-tétraméthyl-2-naphtyl(méthylthio) carbonyl)benzoïque, de point de fusion : 162-163°C.

### EXEMPLE 16

### Acide 5-(5,6,7,8-tétrahydro-5,5,8,8-tétraméthyl-2-naphtoyloxyméthyl)-2-thiophènecarboxylique.

(a) 5-formyl-2-thiophénecarboxylate d'allyle.
   De manière analogue à l'exemple 7(a), à partir de 5,5 g (35 mmoles) d'acide 5-formyl-2-thiophènecarboxylique, on obtient 5 g (73 %) d'ester allylique.
(b) 5-hydroxyméthyl-2-thiophènecarboxylate d'allyle.
   De manière analogue à l'exemple 7(b), à partir de 5 g (25,5 mmoles) de 5-formyl-2-thiophènecarboxylate d'allyle, on obtient 5 g (100 %) du produit attendu, sous forme d'une huile incolore.
(c) 5-(5,6,7,8-tétrahydro-5,5,8,8-tétraméthyl-2-naphtoyloxyméthyl)-2-thiophènecarboxylate d'allyle.
   De manière analogue à l'exemple 1(d), par réaction de 2,5 g (10 mmoles) de chlorure de 5,6,7,8-tétrahydro-5,5,8,8-tétraméthyl-2-naphtoyle avec 1,98 g (10 mmoles) de 5-hydroxyméthyl-2-thiophène carboxylate d'allyle, on obtient 3,7 g (90 %) d'ester allylique, sous forme d'une huile incolore.
(d) Acide 5-(5,6,7,8-tétrahydro-5,5,8,8-tétraméthyl-2-naphtoyloxyméthyl)-2-thiophènecarboxylique.
   De manière analogue à l'exemple 3(c), à partir de 3,4 g (8,4 mmoles) de l'ester obtenu ci-dessus en (c), on obtient 2,7 g (87 %) d'acide 5-(5,6,7,8-tétrahydro-5,5,8,8-tétraméthyl-2-naphtoyloxyméthyl)-2-thiophènecarboxylique, de point de fusion : 155-156°C.

### EXEMPLE 17

### Acide 4-(5,6,7,8-tétrahydro-5,5,8,8-tétraméthyl-2-naphtyloxycarboxamido) benzoïque.

(a) Chloroformiate de 5,6,7,8-tétrahydro-5,5,8,8-tétraméthyl-2-naphtyle.
   Dans un ballon, on introduit 4,1 g (20 mmoles) de 5,6,7,8-tétrahydro-5,5,8,8-tétraméthyl-2-naphtol, 1,2 ml de chloroformiate de trichlorométhyle et 40 ml de benzène. On ajoute goutte à goutte 3 ml (21 mmoles) de triéthylamine et agite à température ambiante douze heures. On verse le milieu réactionnel dans l'eau, extrait avec de l'éther éthylique, décante la phase organique, puis sèche sur sulfate de magnésium et évapore. Le résidu obtenu est purifié par chromatographie sur colonne de silice, élué avec un mélange de dichlorométhane et d'hexane (40-60). Après évaporation des solvants, on recueille 4 g (75 %) du chloroformiate attendu, sous forme d'une huile incolore.
(b) 4-(5,6,7,8-tétrahydro-5,5,8,8-tétraméthyl-2-naphtyloxycarboxamido)benzoate de benzyle.
   Dans un ballon, on introduit 835 mg (3,7 mmoles) de 4-aminobenzoate de benzyle, 300 µl (3,7 mmoles) de pyridine et 20ml de dichlorométhane. On ajoute goutte à goutte une solution de 980 mg (3,7 mmoles) du chloroformiate obtenu ci-dessus en (a) dissous dans 20 ml de dichlorométhane et agite à température ambiante douze heures. On verse le milieu réactionnel dans l'eau, extrait avec de l'éther éthylique, décante la phase organique, puis sèche sur sulfate de magnésium et évapore. Le résidu obtenu est trituré dans le minimum d'hexane et filtré. On recueille 1,6 g (95 %) d'ester benzylique.
(c) Acide 4-(5,6,7,8-tétrahydro-5,5,8,8-tétraméthyl-2-naphtyloxycarboxamido)benzoïque.
   De manière analogue à l'exemple 2(d) à partir de 1,5 g (3,3 mmoles) de l'ester obtenu ci-dessus en (b), on obtient 1 g (83 %) d'acide attendu de point de fusion : 213-214°C.

### EXEMPLE 18

### Acide 4-(5,6,7,8-tétrahydro-5,5,8,8-tétraméthyl-2-naphtylcarbonyldioxy) benzoïque.

(a) 4-(5,6,7,8-tétrahydro-5,5,8,8-tétraméthyl-2-naphtylcarbonyldioxy)benzoate de benzyle.
   De manière analogue à l'exemple 17(b) par réaction de 3,9 g (14,6 mmoles) du chloroformiate préparé, en 17(a) avec 3,33 g (14,6 mmoles) de 4-hydroxybenzoate de benzyle, on obtient 5,3 g (79 %) d'ester benzylique, sous forme d'une huile.
(b) Acide 4-(5,6,7,8-tétrahydro-5,5,8,8-tétraméthyl-2-naphtylcarbonyldioxy)benzoïque.
   De manière analogue à l'exemple 2(d) à partir de 2,9 g (6 mmoles) de l'ester benzylique obtenu ci-dessus en (a), on obtient 1,7 g (77 %) d'acide attendu, de point de fusion : 181-182°C.

### EXEMPLE 19

### Acide 4-[3-(1-adamantyl)-4-méthoxyphénoxycarboxamido]benzoïque.

(a) 3-(1-adamantyl)-4-méthoxyphénol.
   Dans un tricol, on introduit 4,2 g (0,17 mole) de magnésium, un cristal d'iode et 50 ml de THF. On ajoute goutte à goutte une solution de 50 g (0,156 mole) de 2-(1-adamantyl)-4-bromoanisole dans 200 ml de THF et chauffe à reflux pendant trois heures.
   Cette solution est ajoutée goutte à goutte à 0°C à une solution de 19,2 ml (0,172 mole) de borate de triméthyle dans 400 ml d'éther éthylique, puis or agite à température ambiante pendant une heure, ajoute 200 ml d'eau et évapore le milieu réactionnel à sec. On refroidit à 10°C et ajoute successivement une solution de 40 g de chlorure d'ammonium dissous dans 150 ml d'eau puis 100 ml d'eau oxygénée (30 %) et agite à température ambiante pendant six heures. On filtre le solide, le lave à l'eau, sèche sur pentoxyde de phosphore, triture dans 400 ml d'hexane chauffé à reflux et filtre. On recueille 22,9 g (57 %) du phénol attendu de point de fusion : 167-169°C.
(b) Chloroformiate de 3-(1-adamantyl)-4-méthoxyphényle.
   De manière analogue à l'exemple 17(a) à partir de 6,45 g (25 mmoles) de 3-(1-adamantyl)-4-méthoxyphénol, on obtient 5 g (62 %) du chloroformiate attendu de point de fusion : 87-89°C.
(c) 4-[3-(1-adamantyl)-4-méthoxyphénoxycarboxamido]benzoate de benzyle.
   De manière analogue à l'exemple 17(b) par réaction de 2,4 g (7,5 mmoles) de chloroformiate obtenu ci-dessus en (b) avec 1,7 g (7,5 mmoles) de 4-aminobenzoate de benzyle, on obtient 3,2 g (84 %) d'ester benzylique attendu de point de fusion : 208-209°C.
(d) Acide 4-[3-(1-adamantyl)-4-méthoxyphénoxycarboxamido]benzoïque.
   De manière analogue à l'exemple 2(d) à partir de 2 g (3,9 mmoles) de l'ester benzylique obtenu ci-dessus en (c), on obtient 1,2 g (73 %) d'acide attendu de point de fusion : 269-271°C.

### EXEMPLE 20

### Acide 4-[3-(1-adamantyl)-4-méthoxyphénylcarbonyldioxy]benzoïque.

(a) 4-[3-(1-adamantyl)-4-méthoxyphénylcarbonyldioxy]benzoate de benzyle.
   De manière analogue à l'exemple 17(b) par réaction de 2,5 g (7,8 mmoles) de chloroformiate obtenu en 19(b) avec 1,8 g (7,8 mmoles) de 4-hydroxybenzoate de benzyle, on obtient 3,1 g (77 %) d'ester benzylique attendu de point de fusion : 116-118°C.
(b) Acide 4-[3-(1-adamantyl)-4-méthoxyphénylcarbonyldioxy]benzoïque
   De manière analogue à l'exemple 2(d) à partir de 2 g (3,9 mmoles) de l'ester benzylique obtenu ci-dessus en (a), on obtient 1,35 g (83 %) d'acide attendu de point de fusion : 238-239°C.

### EXEMPLE 21

### Acide 4-[1-(5,6,7,8-tétrahydro-5,5,8,8-tétraméthyl-2-naphtoyloxy)éthyl] benzoïque.

(a) 4-acétylbenzoate d'allyle.
   Dans un ballon et sous courant d'azote, on introduit 1,65 g (55 mmoles) d'hydrure de sodium (80 % dans l'huile) et 50 ml de DMF. On ajoute goutte à goutte 8,2 g (50 mmoles) d'acide 4-acétylbenzoïque dissous dans 100 ml de DMF et agite à température ambiante jusqu'à cessation du dégagement gazeux. On introduit ensuite 4,6 ml (55 mmoles) de bromure d'allyle et agite à température ambiante 24 heures. On verse le milieu réactionnel dans l'eau, extrait avec de l'éther éthylique, décante la phase organique puis sèche sur sulfate de magnésium et évapore. le résidu obtenu est purifié par chromatographie sur colonne de silice en éluant avec un mélange de dichlorométhane et d'hexane (80-20). Après évaporation des solvants, on recueille 7,2 g (71 %) d'ester allylique, sous forme d'une huile incolore.
(b) 4-(1-hydroxyéthyl)benzoate d'allyle.
   Dans un ballon, on introduit 3,1 g (15 mmoles) de l'ester allylique obtenu ci-dessus en (a), 20 ml d'alcool méthylique et 20 ml de THF. On ajoute par petites quantités, tout en refroidissant avec un bain de glace, 265 mg (7,5 mmoles) de borohydrure de sodium et agite à température ambiante une heure. On verse le milieu réactionnel dans l'eau, extrait avec de l'éther éthylique, décante la phase organique puis, sèche sur sulfate de magnésium et évapore. On recueille 3,1 g (100 %) du produit attendu sous forme d'une huile incolore.
(c) 4-[1-(5,6,7,8-tétrahydro-5,5,8,8-tétraméthyl-2-naphtoyloxy) éthyl]benzoate d'allyle.
   De manière analogue à l'exemple 4(c) par réaction de 3 g (12,9 mmoles) d'acide 5,6,7,8-tétrahydro-5,5,8,8-tétraméthyl-2-naphtoïque avec 2,65 g (12,9 mmoles) de 4-(1-hydroxyéthyl)benzoate d'allyle, on obtient 2,8 g (52 %) de l'ester allylique attendu, sous forme d'une huile légèrement jaune.
(d) Acide 4-[1-(5,6,7,8-tétrahydro-5,5,8,8-tétraméthyl-2-naphtoyloxy)éthyl]benzoïque.
   De manière analogue à l'exemple 3(c) à partir de 2,8 g (6,7 mmoles) de l'ester allylique obtenu ci-dessus en (c), on obtient 2 g (79 %) d'acide attendu de point de fusion : 149-150°C.

### EXEMPLE 22

### Acide 4-[[1-(5,6,7,8-tétrahydro-5,5,8,8-tétraméthyl-2-naphtyl) éthyloxy]carbonyl]benzoïque

(a) 4-[[1-(5,6,7,8-tétrahydro-5,5,8,8-tétraméthyl-2-naphtyl) éthyloxy]carbonyl]benzoate d'allyle.
   De manière analogue à l'exemple 4(c) par réaction de 2,1 g (10 mmoles) d'acide 4-(allyloxycarbonyl)benzoïque avec 2,3 g (10 mmoles) de 1-(5,6,7,8-tétrahydro-5,5,8,8-tétraméthyl-2-naphtyl)éthanol, on obtient 3,2 g (76 %) de l'ester allylique attendu sous forme d'une huile incolore.
(b) Acide 4-[[1-(5,6,7,8-tétrahydro-5,5,8,8-tétraméthyl-2-naphtyl) éthyloxy]carbonyl]benzoïque.
   De manière analogue à l'exemple 3(c) à partir de 2,8 g (6,6 mmoles) de l'ester allylique obtenu ci-dessus en (a), on obtient 2,3 g (92 %) d'acide attendu de point de fusion : 176-177°C.

### EXEMPLE 23

### Acide 4-[3-(1-adamantyl)-3-méthoxyphénylacétamido]benzoïque

(a) 3-(1-adamantyl)-4-hydroxyphénylacétate de méthyle.
   De manière analogue à l'exemple 10(a) par réaction de 4,6 g (30 mmoles) de 1-adamantanol avec 5 g (30 mmoles) de 4-hydroxyphénylacétate de méthyle, on obtient 4,9 g (54 %) de 3-(1-adamantyl)-4-hydroxyphénylacétate de méthyle de point de fusion : 185-186°C.
(b) 3-(1-adamantyl)-4-méthoxyphénylacétate de méthyle.
   De manière analogue à l'exemple 10(b) par réaction de 3 g (10 mmoles) d'ester obtenu ci-dessus en (a) avec 750µl (12 mmoles) d'iodure de méthyle, on obtient 1,3 g (40 %) de l'ester attendu de point de fusion : 70-71°C.
(c) Acide 3-(1-adamantyl)-4-méthoxyphénylacétique.
   De manière analogue à l'exemple 12(b) à partir de 1,27 g (4 mmoles) de l'ester obtenu ci-dessus en (b), on obtient 1,12 g (93 %) d'acide 3-(1-adamantyl)-4-méthoxyphénylacétique de point de fusion : 223-224°C.
(d) Chlorure de 3-(1-adamantyl)-4-méthoxyphénylacétyle.
   De manière analogue à l'exemple 2(b) à partir de 1,1 g (3,7 mmoles) de l'acide obtenu ci-dessus en (c), on obtient après lavage dans l'hexane 860 mg (74 %) de chlorure d'acide de point de fusion : 78-79°C.
(e) 4-[3-(1-adamantyl)-4-méthoxyphénylacétamido]benzoate de méthyle.
   De manière analogue à l'exemple 1(d) par réaction de 3,8 g (11,9 mmoles) de chlorure de 3-(1-adamantyl)-4-méthoxyphénylacétyle avec 1,81 g (12 mmoles) de 4-aminobenzoate de méthyle, on obtient 3,2 g (63 %) de l'ester méthylique attendu de point de fusion : 146-147°C.
(f) Acide 4-[3-(1-adamantyl)-4-méthoxyphénylacétamido]benzoïque.
   De manière analogue à l'exemple 12(b) à partir de 3 g (6,9 mmoles) de l'ester obtenu ci-dessus en (e), on obtient 2,2 g (76 %) d'acide attendu de point de fusion : 238-240°C.

### EXEMPLE 24

### Acide 4-(5,6,7,8-tétrahydro-5,5,8,8-tétraméthyl-2-naphtoylformamido) benzoïque

(a) 4-(5,6,7,8-tétrahydro-5,5,8,8-tétraméthyl-2-naphtoylformamido)benzoate d'allyle.
   De manière analogue à l'exemple 1(d) par réaction de 5,6 g (20 mmoles) de chlorure de 5,6,7,8-tétraméthyl-2-naphtylglyoxyloyle avec 3,6 g (20 mmoles) de 4-aminobenzoate d'allyle, on obtient 3,5 g (42 %) de l'ester attendu sous forme d'une huile légèrement jaune.
(b) Acide 4-(5,6,7,8-tétrahydro-5,5,8,8-tétraméthyl-2-naphtoylformamido)benzoïque.
   Dans un ballon et sous courant d'azote, on introduit 105 mg (3,5 mmoles) d'hydrure de sodium (80 % dans l'huile) et 5 ml de THF. On ajoute ensuite goutte à goutte 530 µl (3,5 mmoles) de malonate de diéthyle et agite jusqu'à cessation du dégagement gazeux. Cette solution est introduite goutte à goutte dans un mélange de 1,47 g (3,5 mmoles) de l'ester allylique préparé à l'exemple 24 (a) et 210 mg (0,18 mmoles) de tétrakis(triphénylphosphine) palladium(0) et on agite à température ambiante pendant une heure. On verse le milieu réactionnel dans l'eau, extrait avec de l'éther éthylique, décante la phase organique, sèche sur sulfate de magnésium et évapore. Le résidu obtenu est purifié par chromatographie sur colonne de silice élué avec un mélange de dichlorométhane et d'éther éthylique (95-5). Après évaporation des solvants, on recueille 1,1 g (85 %) d'acide de point de fusion : 216-217°C.

### EXEMPLE 25

### Acide 4-(α-hydroxy-5,6,7,8-tétrahydro-5,5,8,8-tétraméthyl-2-naphtylacétamido)benzoïque

(a) 4-(α-hydroxy-5,6,7,8-tétrahydro-5,5,8,8-tétraméthyl-2-naphtylacétamido)benzoate d'allyle.
   De manière analogue à l'exemple 5 (b) à partir de 1,47 g (3,5 mmoles) de l'ester préparé à l'exemple 24(a), on obtient 1,3 g (90 %) d'ester attendu, sous forme d'une huile incolore.
(b) Acide 4-(α-hydroxy-5,6,7,8-tétrahydro-5,5,8,8-tétraméthyl-2-naphtylacétamido)benzoïque.
   De manière analogue à l'exemple 24(b) à partir de 1,3 g (3,1 mmoles) de l'ester préparé à l'exemple 25(a), on obtient 1,1 g (93 %) d'acide 4-(α-hydroxy-5,6,7,8-tétrahydro-5,5,8,8-tétraméthyl-2-naphtyl acétamido)benzoïque, de point de fusion : 220-221°C.

### EXEMPLE 26

### Acide 4-(5,6,7,8-tétrahydro-5,5,8,8-tétraméthyl-2-naphtylcarbamoyl méthyl)benzoïque

(a) Acide 4-(tert-butoxycarbonylméthyl)benzoïque.
   Dans un tricol, on introduit 11,4 g (0,170 mole) de Zinc en poudre et 50 ml de THF, on ajoute 2,2 ml de chlorure de triméthylsilane et agite 15 minutes puis introduit goutte à goutte 28 ml (0,174 mode) de 2-bromoacétate de tert-butyle et agite 30 minutes. On refroidit à 0°C (bain de glace), introduit 5 g (4,3 mmoles) de tétrakis(triphénylphosphine)palladium(0), 10,8 g (43,5 mmoles) d'acide 4-iodobenzoïque, 50 ml de HMPA et chauffe à 40°C pendant une heure. On verse le milieu réactionnel dans l'eau, extrait avec de l'éther éthylique, décante la phase organique, sèche sur sulfate de magnésium et évapore. Le résidu obtenu est purifié par chromatographie sur colonne de silice, élué avec un mélange de dichlorométhane et d'éther éthylique (95-5). Après évaporation des solvants, on recueille 8,1 g (80 %) du produit attendu de point de fusion : 132-133°C.
(b) 4-(allyloxycarbonyl)phénylacétate de tert-butyle.
   De manière analogue à l'exemple 7 (a) à partir de 7,9 g (33,4 mmoles) d'acide 4-(tert-butoxycarbonylméthyl) benzoïque, on obtient 8 g (87 %) de l'ester allylique sous forme d'une huile incolore.
(c) Acide 4-(allyloxycarbonyl)phénylacétique.
   De manière analogue à l'exemple 1 (e) à partir de 7,4 g (26,8 mmoles) du di-ester obtenu ci-dessus en (b), on obtient 5,8 g (100 %) du mono acide attendu de point de fusion : 68-69°C.
(d) 4-(5,6,7,8-tétrahydro-5,5,8,8-tétraméthyl-2-naphtylcarbamoylméthyl)benzoate d'allyle
   De manière analogue à l'exemple 4(c) par réaction de 2 g (10 mmoles) de 5,6,7,8-tétrahydro-5,5,8,8-tétraméthyl-2-naphtylamine avec 2,2 g (10 mmoles) d'acide 4-(allyloxycarbonyl)phénylacétique, on obtient 3,7 g (92 %) de l'ester attendu de point du fusion 49-50°C.
(e) Acide 4-(5,6,7,8-tétrahydro-5,5,8,8-tétraméthyl-2 naphtylcarbamoylméthyl)benzoïque.
   De manière analogue à l'exemple 24 (b) à partir de 3,5 g (8,6 mmoles) de l'ester obtenu ci-dessus en (d), on obtient 1,4 g (47 %) d'acide attendu de point de fusion 249-250°C.

### EXEMPLE 27

### Acide 2-hydroxy-4-(5,6,7,8-tétrahydro-5,5,8,8-tétraméthyl-2-naphtylacétoxy)benzoïque

(a) 2-hydroxy-4-(5,6,7,8-tétrahydro-5,5,8,8-tétraméthyl-2-naphtylacétoxy)benzoate de benzyle
   De manière analogue à l'exemple 1(d) par réaction de 2,44 g (10 mmoles) de 2,4-di-hydroxybenzoate de benzyle avec 2,6 g (10 mmoles) de chlorure de 5,6,7,8-tétrahydro-5,5,8,8-tétraméthyl-2-naphtylacétyle, on obtient 3,9 g (83 %) de l'ester attendu sous forme d'une huile incolore.
(b) Acide 2-hydroxy-4-(5,6,7,8-tétrahydro-5,5,8,8-tétaméthyl-2-naphtylacétoxy)benzoïque
   De manière analogue à l'exemple 2(d), à partir de 3,4 g (7,2 mmoles) de l'ester obtenu ci-dessus en (a) on obtient 2,5 g (91 %) d'acide de point de fusion : 163-164°C.

### EXEMPLE 28

### Acide 4-(5,6,7,8-tétrahydro-5,5,8,8-tétaméthyl-2-naphtyloxycarbonyl méthyl)benzoïque

(a) 4-(benzyloxycarbonyl)phénylacétate de tert-butyle.
   De manière analogue à l'exemple 7 (a) par réaction de 3 g (12,7 mmoles) d'acide 4-(tert-butoxycarbonylméthyl)benzoïque préparé à l'exemple 29(a) avec 1,6 ml (14 mmoles) de bromure de benzyle, on obtient 3,2 g (77 %) d'ester benzylique sous forme d'une huile légèrement jaune.
(b) Acide 4-(benzyloxycarbonyl)-phénylacétique.
   De manière analogue à l'exemple 1(e) à partir de 3,1 g (9,5 mmoles) du di-ester précédent, on obtient 2,3 g (90 %) du mono-ester attendu de point de fusion : 107-108°C.
(c) 4-(5,6,7,8-tétrahydro-5,5,8,8-tétraméthyl-2-naphtyloxycarbonylméthyl)benzoate de benzyle.
   De manière analogue à l'exemple 4(c) par réaction de 1,97 g (7,3 mmoles) avec 1,48 g (7,3 mmoles) de 5,6,7,8-tétrahydro-5,5,8,8-tétraméthyl-2-naphtol, on obtient 2,5 g (75 %) du produit attendu sous forme d'une huile incolore.
(d) Acide 4-(5,6,7,8-tétrahydro-5,5,8,8,-tétraméthyl-2-naphtyloxycarbonylméthyl)benzoïque
   De manière analogue à l'exemple 2(d) à partir de 2,43 g (5,2 mmoles) de l'ester benzylique précédent, on obtient 1,1 g (58 %) d'acide de point de fusion : 131-132°C.

### EXEMPLE 29

### Acide 4-(N-méthyl-5,6,7,8-tétrahydro-5,5,8,8-tétraméthyl-2-naphtylacétamido)benzoïque

(a) 4-(N-méthyl-5,6,7,8-tétrahydro-5,5,8,8-tétraméthyl-2-naphtylacétamido)benzoate de méthyle.
   De manière analogue à l'exemple 4(c) par réaction de 2,46 g (10 mmoles) d'acide 5,6,7,8-tétrahydro-5,5,8,8-tétraméthyl-2-naphtylacétique avec 1,65 g (10 mmoles) de 4-méthylaminobenzoate de méthyle, on obtient 2,1 g (54 %) de produit attendu sous forme d'une huile incolore.
(b) Acide 4-(N-méthyl-5,6,7,8-tétrahydro-5,5,8,8-tétraméthyl-2-naphtylacétamido)benzoïque.
   Dans un ballon, on introduit 1,8 g (4,6 mmoles) de l'ester obtenu ci-dessus en (a), 100 ml de soude méthanolique 2N et 50 ml de THF. On agite à température ambiante pendant une heure, évapore à sec, reprend par l'eau, extrait avec de l'éther éthylique et décante la phase aqueuse. On acidifie à pH 1 la phase aqueuse avec de l'acide chlorhydrique concentré, extrait avec de l'éther éthylique, décante la phase organique, sèche sur sulfate de magnésium et évapore. On recueille 1,6 g (94 %) d'acide attendu au point de fusion : 192-193°C.

### EXEMPLE 30

### Acide 4-(α-fluoro-5,6,7,8-tétrahydro-5,5,8,8-tétraméthyl-2-naphtylacétamido)benzoïque

(a) α-hydroxy-5,6,7,8-tétrahydro-5,5,8,8-tétraméthyl-2-naphtylacétate d'éthyle.
   De manière analogue à l'exemple 5(b) à partir de 10,3 g (35,7 mmoles) de 5,6,7,8-tétrahydro-5,5,8,8-tétraméthyl-2-naphtylglyoxylate d'éthyle, on obtient 7,5 g (73 %) de l'alcool attendu sous forme d'une huile légèrement jaune.
(b) α-fluoro-5,6,7,8-tétrahydro-5,5,8,8-tétraméthyl-2-naphtylacétate d'éthyle.
   Dans un ballon, on introduit 2,9 g (10 mmoles) de l'alcool précédent et 50 ml de dichlorométhane. Sous courant d'azote et à -78°C, on ajoute goutte à goutte une solution de 1,3 ml de trifluorure de diéthylaminosulfure (DAST) dans 30 ml de dichlorométhane et laisse remonter la température à 25 °C. On verse le milieu réactionnel dans l'eau, extrait avec de l'éther éthylique, décante la phase organique, sèche sur sulfate de magnésium et évapore. Le résidu est purifié par chromatographie sur colonne de silice, élué avec un mélange de dichlorométhane et d'hexane (20-80). Après évaporation des solvants, on recueille 2,3 g (79 %) du composé fluoré attendu sous forme d'une huile incolore.
(c) Acide α-fluoro-5,6,7,8-tétrahydro-5,5,8,8-tétraméthyl-2-napthylacétique.
   Dans un ballon, on introduit 2,3 g (7,8 mmoles) de l'ester fluoré précédent, 100 ml d'acide chlorhydrique (10 %) et 50 ml de THF. On chauffe à reflux pendant 24 heures, évapore à sec, reprend par l'éther, lave la phase organique à l'eau sèche sur sulfate de magnésium et évapore. On triture le résidu obtenu dans l'hexane et après filtration recueille 1,5 g (75 %) d'acide de point de fusion : 127-128°C.
(d) 4-(α-fluoro-5,6,7,8-tétrahydro-5,5,8,8-tétraméthyl-2-naphtylacétamido)benzoate d'allyle.
   De manière analogue à l'exemple 4(c) par réaction de 1,35 g (5,1 mmoles) d'acide α-fluoro-5,6,7,8-tétrahydro-5,5,8,8-tétraméthyl-2-naphtylacétique avec 920 mg (5,1 mmoles) de 4-aminobenzoate d'allyle, on obtient 1,7 g (81 %) d'ester sous forme d'une huile.
(e) Acide 4-(α-fluoro-5,6,7,8-tétrahydro-5,5,8,8-tétraméthyl-2-napthylacétamido)benzoïque.
   De manière analogue à l'exemple 3(c) à partir de 1,56 g (3,7 mmoles) de l'ester précédent, on recueille 1,3 g (93 %) d'acide 4-(α -fluoro-5,6,7,8-tétrahydro-5,5,8,8-tétraméthyl-2-naphtylacétamido) benzoïque de point de fusion : 213-214°C.

### EXEMPLE 31

### Acide 4-[3-(1-adamantyl)-4-méthoxyphényluréido]benzoïque

(a) 3-(1-adamantyl)-4-méthoxy-(N-trifluoroacétyl)aniline.
   A une solution de 20,6 g (70 mmoles) d'acide 3-(1-adamantyl)-4-méthoxybenzoïque dans 200 ml de tétrahydrofuranne à 0°C on additionne goutte à goutte successivement 11,70 ml (84 mmoles) de triéthylamine dans 10 ml de tétrahydrofuranne et 8,70 ml (91 mmoles) de chloroformate d'éthyle dans 10 ml de tétrahydrofuranne. Le milieu réactionnel est agité pendant 40 minutes à 0°C, puis on ajoute à ce milieu 6,83 g (105 mmoles) d'azoture de sodium en solution dans 30 ml d'eau et laisse agiter à 0°C pendant 2 heures. Le milieu réactionnel est versé dans l'eau glacé, extrait par du dichlorométhane, lavé à l'eau, séché sur sulfate de magnésium et évaporé. Le résidu est dissous dans 400 ml de dichlorométhane, on ajoute 7,3 ml (0,094 modes) d'acide trifluoroacétique et chauffe à reflux pendant 30 heures. On verse le milieu réactionnel dans un solution saturée en bicarbonate de sodium, sèche sur sulfate de magnésium et évapore. Le résidu est chromatographié sur silice avec le mélange dichlorométhane/hexane (30 : 70) pour conduire à 14,1 g (57 %) de 3-(1-adamantyl)-4-méthoxy-(N-trifluoroacétyl)aniline de point de fusion 166-167°C.
(b) 3-(1-adamantyl)-4-méthoxyaniline.
   11,03 g (31,2 mmoles) de 3-(1-adamantyl)-4-méthoxy-(N-trifluoro acétyl)aniline en solution dans 40 ml de méthanol et 160 ml d'eau sont traités par 7,12 g (51,5 mmoles) de potasse. Le milieu réactionnel est chauffé à 50°C pendant 20 heures puis 80°C pendant 18 heures. Après évaporation à sec, le résidu est repris par de l'éther éthylique, on filtre l'insoluble, sèche sur sulfate de magnésium et évapore. Le solide est lavé dans l'hexane pour conduire après séchage à 7,44 g (93 %) de 3-(1-adamantyl)-4-méthoxyaniline, de point de fusion 140-141°C.
(c) 4-[3-(1-adamantyl)-4-méthoxyphényluréido]benzoate de méthyle.
   Une solution de 2,81 g (15,6 mmoles) d'acide 4-(méthoxycarbonyl)benzoïque dans 30 ml de tétrahydrofuranne est placée à 0°C puis est traitée par 2,61 ml (18,7 mmoles) de triéthylamine et 1,94 ml (20,3 mmoles) de chloroformiate d'éthyle et est laissée sous agitation 1 heure à 0°C. On ajoute à ce milieu une solution de 1,52 g (23,4 mmoles) d'azoture de sodium dans 10 ml d'eau, laisse agiter à 0°C pendant 3 heures puis verse le milieu réactionnel dans l'eau glacée et extrait avec de l'éther éthylique. La phase organique est lavée à l'eau, séchée sur sulfate de magnésium et évaporée. Le résidu est mis en solution dans 30 ml de toluène et chauffé à 100°C pendant 1,5 heures puis est refroidi à température ambiante. On ajoute alors goutte à goutte à cette solution précédente 4,82 g (18,7 mmoles) de 3-(1-adamantyl)-4-méthoxyaniline et agite à 50°C pendant 1,5 heures. On évapore à sec, reprend le solide par l'éther éthylique, sèche sur sulfate de magnésium et évapore. Le résidu est recristallisé dans l'acétate d'éthyle pour conduire à 6,16 g (91 %) de 4-[3-(1-adamantyl)-4-méthoxyphényluréido]benzoate de méthyle de point de fusion 223-224°C.
(d) Acide 4-[3-(1-adamantyl)-4-méthoxyphényluréido]benzoïque.
   A une solution de 6,08 g (14 mmoles) de 4-[3-(1-adamantyl) -4-méthoxyphényluréido]benzoate de méthyle dans 75 ml de méthanol, on ajoute 6 g (0,15 moles) de soude et agite à température ambiante pendant 3 heures puis chauffe à reflux 3,5 heures. Le mélange réactionnel est évaporé puis est repris par 100 ml d'eau et acidifié à pH 1. Le solide est filtré, rincé à l'eau, séché et recristallisé dans un mélange tétrahydrofuranne/hexane pour conduire à 2,78 g (87 %) d'acide 4-[3-(1-adamantyl)-4-méthoxy phényluréido]benzoïque, de point de fusion 279-282°C.

### EXEMPLE 32

### Acide 2-hydroxy-4-(5,6,7,8-tétrahydro-5,5,8,8-tétraméthyl-2-naphtoylméthyloxy)benzoïque :

a) 2-hydroxy-4-(5,6,7,8-tétrahydro-5,5,8,8-tétraméthyl-2-naphtoylméthyloxy)benzoate d'allyle :
   Dans un ballon on introduit 0,01 mole d'hydrure de sodium (80% dans l'huile) et 20 ml de DMF. On ajoute goutte à goutte une solution de 1,9 g (0,01 mole) de 2,4-dihydroxybenzoate d'allyle dissous dans 50 ml de DMF et agite jusqu'à cessation du dégagement gazeux. On introduit ensuite une solution de 3,1 g (0,01 mole) de 2-(2'-bromoacéto) 5,6,7,8-tétrahydro-5,5,8,8-tétraméthylnaphtone dans 20 ml de DMF.
   Après agitation 4 heures à température ambiante, extraction et chromatographie sur colonne de silice on obtient 3,4 g (81%) d'ester allylique sous forme d'une huile légèrement jaune.
b) Acide 2-hydroxy-4-(5,6,7,8-tétrahydro-5,5,8,8-tétraméthyl-2-naphtoylméthyloxy)benzoïque :
   De manière analogue à l'exemple 3(c) à partir de 3,4 g (8 mmoles) de l'ester allylique précédent, on obtient 640 mg (21%) d'acide attendu de point de fusion 202-3°C.

### EXEMPLE 33

### Acide 4-(α-méthylène-5,6,7,8-tétrahydro-5,5,8,8-tétraméthyl-2-naphtylacétamido)benzoïque :

a) 4-(α-méthylène-5,6,7,8-tétrahydro-5,5,8,8-tétraméthyl-2-naphtylacétamido)benzoate de méthyle :
   De manière analogue à l'exemple 4(c) par réaction de 4,6 g (18 mmoles) d'acide α-méthylène-5,6,7,8,-tétrahydro-5,5,8,8-tétraméthyl-2-naphtylacétique avec 2,7 g (18 mmoles) de 4-aminobenzoate de méthyle, on obtient 2,4 g (34%) de l'ester attendu sous forme d'une huile incolore.
b) Acide 4-(α-méthylène-5,6,7,8-tétrahydro-5,5,8,8-tétraméthyl-2-naphtylacétamido)benzoïque :
   De manière analogue à l'exemple 12(b) à partir de 2,2 g (5,6 mmoles) de l'ester méthylique précédent, on obtient 1,6 g (76%) de l'acide attendu de point de fusion 207-8°C.

### EXEMPLE 34

### Acide 4-[2-(5,6,7,8-tétrahydro-5,5,8,8-tétraméthyl-2-naphtyl)propionamido]benzoïque :

Dans un réacteur, on introduit 650 mg (1,7 mmole) de l'acide 4-(α-méthylène-5,6,7,8-tétrahydro-5,5,8,8-tétraméthyl-2-naphtylacétamido)benzoïque, 100 mg de palladium sur charbon (10%) et 100 ml de Dioxanne. On hydrogène à température ambiante et sous une pression de 7 bars pendant 2 h, filtre le catalyseur et évapore le filtrat. Le résidu obtenu est trituré dans le minimum d'éther éthylique, filtré, séché. On obtient 580 mg (89%) d'acide de point de fusion 195-6°C.

### EXEMPLE 35

### 2-hydroxy-4-(5,6,7,8-tétrahydro-5,5,8,8-tétraméthyl-2-naphtoylformamido)benzoate de méthyle :

Dans un ballon, on introduit 6,7 g (40 mmoles) de 2-hydroxy-4-aminobenzoate de méthyle, 10 ml (0,12 mole) de pyridine et 50 ml de THF. On ajoute goutte à goutte une solution de 10,5 g (40 mmoles) de chlorure 5,6,7,8-tétrahydro-5,5,8,8-tétraméthyl-2-naphtylglyoxyloyle préparé en 1(c) dans 100 ml de THF et agite à température ambiante 4 h. On verse le milieu réactionnel dans l'eau, extrait avec de l'éther éthylique, décante la phase organique, sèche sur sulfate de magnésium, évapore. Le résidu obtenu est purifié par chromatographie sur colonne de silice élué avec un mélange de dichlorométhane et d'hexane (30-70). Après évaporation des solvants, on recueille 7,3 g (45%) d'ester méthylique de point de fusion 146-7°C.

### EXEMPLE 36

### Acide 2-hydroxy-4-(α-hydroxy-5,6,7,8-tétrahydro-5,5,8,8-tétraméthyl-2-naphtylacétamido)benzoïque :

a) 2-hydroxy-4-(α-hydroxy-5,6,7,8-tétrahydro-5,5,8,8-tétraméthyl-2-naphtylacétamido)benzoate de méthyle :
   Dans un ballon et sous courant d'azote on introduit 2,8 g (7 mmoles) de l'ester méthylique obtenu à l'exemple 35, 50 ml de THF et 50 ml d'alcool méthylique. Tout en refroidissant avec un bain de glace, on ajoute par petites quantités 130 mg (3,5 moles) de borohydrure de sodium et agite à température ambiante pendant 2 h.
   On verse le milieu réactionnel dans l'eau, extrait avec de l'éther éthylique, décante la phase organique, sèche sur sulfate de magnésium, évapore. On recueille 2.8g (100%) d'ester méthylique de point de fusion 168-9°C
b) Acide 2-hydroxy-4-(α-hydroxy-5,6,7,8-tétrahydro-5,5,8,8-tétraméthyl-2-naphtylacétamido)benzoïque :
   De manière analogue à l'exemple 12(b) à partir de 2,8 g (6,8 mmoles) de l'ester méthylique précédent, on obtient 2 g de l'acide attendu de point de fusion 178-9°C.

### EXEMPLE 37

### Acide 2-hydroxy-4-(3,5-di-tert-butyl-4-hydroxybenzoylméthyloxy)benzoïque :

a) 2'-bromo-3,5-di-tert-butyl-4-hydroxyacétophénone:
   Dans un ballon on introduit 2,5 g (0,01 mole) de 3,5 ditert-butyl-4 hydroxacétophénone, 25 ml d'éther éthylique et 25 ml de dioxanne. On ajoute goutte à goutte 0,01 mole de brome et agite à température ambiante pendant 1 heure. Après extraction et évaporation des solvants ou chromatographie sur colonne de silice. On obtient après évaporation du solvant d'élution 1,5 g (46 %) du dérivé bromé de point de fusion 103-4 °C.
b) 2-hydroxy-4-(3,5-di-tert-butyl-4-hydroxybenzoylméthyloxy)benzoate de benzyle:
   De manière analogue à l'exemple 32(a) par réaction de 1,5 g (4,6 mmoles) du dérivé bromé précèdent avec 1,2 g (4,9 mmoles) de 2,4-dihydroxybenzoate de benzyle, on obtient 1,6 g (71%) d'ester benzylique de point de fusion 123-4°C.
c) Acide 2-hydroxy-4-(3,5-di-tert-butyl-4-hydroxybenzoylméthyloxy)benzoïque:
   Dans un reacteur, on introduit 1,6 g (3,2 mmoles) de l'ester précédent, 260 mg de palladium sur charbon (10%) et 60 ml de dioxanne. On hydrogène à température ambiante et sous une pression de 7 bars pendant 2 h, filtre le catalyseur et évapore le filtrat. Le résidu obtenu est trituré dans l'hexane et séché, on obtient 1,1 g (86%) d'acide attendu de point de fusion 166-7°C.

### EXEMPLE 38

### 2-hydroxy-4-(5,6,7,8-tétrahydro-5,5,8,8-tétraméthyl-2-naphtoylméthyloxy)benzaldéhyde :

Dans un ballon, on introduit 12.4 g (0.04 mole) de 2-(2'-bromoacéto) -5,6,7,8-tétrahydro-5,5,8,8-tétraméthyl naphtone, 5,5 g (0,04 mole) de 2,4-dihydroxybenzaldéhyde, 5,5 g de carbonate de potassium et 400 ml de méthyléthylcétone. On chauffe à reflux 1 h, titre et évapore le filtrat. Le résidu obtenu est purifié par chromatographie sur colonne de silice, élué avec du dichlorométhane. Après évaporation des solvants, on recueille 9,9 g (67%) de l'aldéhyde attendu de point de fusion 111-2°C.

### EXEMPLE 39

### 1-(5,6,7,8-tétrahydro-5,5,8,8-tétraméthyl-2-naphtyl)-2-(3-hydroxy-4-méthylphénoxy)éthanone :

Dans un réacteur, on introduit 1 g (2,7 mmoles) de 2-hydroxy-4-(5,6,7,8-tétrahydro-5,5,8,8-tétraméthyl-2-naphtoylméthyloxy)benzaldéhyde, 100 mg de palladium sur charbon (5%) et 50 ml de dioxanne. On hydrogène à température ambiante et sous une pression de 3 bars, filtre le catalyseur et évapore le filtrat. Le résidu obtenu est purifié par chromatographie sur colonne de silice élué avec un mélange d'hexane et d'éther éthylique (70-30). Après évaporation des solvants, on recueille 600 mg du produit attendu de point de fusion 144-5°C.

### EXEMPLE 40

### 2,6-dihydroxy-4-(5,6,7,8-tétrahydro-5,5,8,8-tétraméthyl-2-naphtoylméthyloxy)benzoate de méthyle :

De manière analogue à l'exemple 38, par réaction de 9,3 g (0,03 mole) de 2-(2'-bromoacéto)-5,6,7,8-tétrahydro-5,5,8,8-tétraméthylnaphtone avec 5,5 g (0,03 mole) de 2,4,6-trihydroxybenzoate de méthyle, on obtient 7,2 g (57%) d'ester méthylique de point de fusion 162-3°C.

### EXEMPLE 41

### N-éthyl-4-(3-tert-butyl-4-méthoxybenzoyloxyméthyl)benzamide :

a) Chlorure de 4-(3-tert-butyl-4-méthoxybenzoyloxyméthyl)benzoyle :
   De manière analogue à l'exemple 1(c) à partir de 3,4 g (0,01 mole) d'acide 4-((3-tert-butyl-4-méthoxybenzoyloxyméthyl)benzoïque préparé à l'exemple 8, on obtient 3,6 g (100%) de chlorure d'acide brut qui est utilisé tel quel pour la suite de la synthèse.
b) N-éthyl-4-(3-tert-butyl-4-méthoxybenzoyloxyméthyl)benzamide :
   Dans un ballon, on introduit 12 ml d'éthylamine et 50 ml de THF, ajoute goutte à goutte une solution de 1,2 g (3,3 mmoles) de chlorure d'acide précédent dissous dans 30 ml de THF et agite à température ambiante 4 h. On verse ce milieu réactionnel dans l'eau, extrait avec de l'éther éthylique, décante la phase organique, sèche sur sulfate de magnésium, évapore. Le résidu obtenu est trituré dans un mélange d'hexane et d'éther éthylique (90-10) et filtré. On recueille 740 mg (67%) d'amide de point de fusion 107-8°C.

### EXEMPLE 42

### Acide 2-hydroxy-4-(3-tert-butyl-4-méthoxybenzoylméthyloxy)benzoïque :

a) 3-tert-butyl-4-méthoxyacétophénone :
   Dans un tricol et sous courant d'azote on introduit 22,6 g (0,1 mole) de chlorure de 3-tert-butyl-4-méthoxybenzoyle, 30 ml de HMPA et ajoute successivement 14 ml (0,1 mole) de tétraméthylétain et 43 mg de benzyl(chloro)bis-(triphénylphosphine)palladium(II).
   On chauffe à 80°C durant 4 h et agite à température ambiante pendant 12 h. On verse le milieu réactionnel dans l'eau, extrait avec de l'éther éthylique, décante la phase organique, sèche sur sulfate de magnésium, évapore. Le résidu obtenu est purifié par chromatographie sur colonne de silice, élué avec un mélange d'hexane et de dichlorométhane (50-50). Après évaporation des solvants, on recueille 11,5 g (58%) du produit attendu de point de fusion 68-9°C.
b) 2'-bromo-3-tert-butyl-4-méthoxyacétophénone :
   De manière analogue à l'exemple 37(a) à partir de 8,2 g (0,04 mole) de 3-tert-butyl-4-méthoxyacétophénone, on obtient 8,7 g (76%) du dérivé bromé sous forme d'une huile légèrement jaune.
c) 2-hydroxy-4-(3-tert-butyl-4-méthoxybenzoylméthyloxy)benzoate de benzyle :
   De manière analogue à l'exemple 38 par réaction de 8,7 g (0,03 mole) du bromé précédent avec 7,5 g (0,03 mole) de 2,4-dihydroxybenzoate de benzyle, on obtient 11 g (80%) d'ester benzylique de point de fusion 98-9°C.
d) Acide 2-hydroxy-4-(3-tert-butyl-4-méthoxybenzoylméthyloxy)benzoïque :
   De manière analogue à l'exemple 37(c) à partir de 2 g (4,4 mmoles) de l'ester précédent, on obtient 750 mg (48%) de l'acide attendu de point de fusion 170-1°C.

### EXEMPLE 43

### 4-(3-tert-butyl-4-méthoxybenzoyloxyméthyl)benzoate de méthyle :

Dans un ballon, on introduit 5 ml de méthanol, 460 µl de triethylamine et 50 ml de THF. On ajoute goutte à goutte une solution de 1,2 g (3,3 mmoles) de chlorure de 4-(3-tert-butyl-4-méthoxybenzoyloxyméthyl)benzoyle dissous dans 30 ml de THF et agite à température ambiante 4 h.

On évapore à sec, reprend le résidu par l'eau et l'éther éthylique, décante la phase organique, sèche sur sulfate de magnésium, évapore. Le résidu obtenu est purifié par chromatographie sur silice, élué avec un mélange d'hexane et de dichlorométhane (50-50). Après évaporation des solvants, on recueille 580 mg (59%) de l'ester attendu de point de fusion 76-7°C.

### EXEMPLE 44

### N-pyrrolidinyl-4-(3-tert-butyl-4-méthoxybenzoyloxyméthyl)benzamide

Dans un ballon, on introduit 760 µl (5,5 mmoles) de triethylamine, 450 µl (5,5 mmoles) de pyrrolidine et 50 ml de THF. On ajoute goutte à goutte 1,8 g (5 mmoles) de chlorure de 4-(3-tert-butyl-4-méthoxybenzoyloxyméthyl)benzoyle dissous dans 50 ml de THF et agite à température ambiante 4 h. On verse le milieu réactionnel dans l'eau extrait avec de l'éther éthylique, décante la phase organique, sèche sur sulfate de magnésium, évapore.

Le résidu est purifié par chromatographie sur colonne de silice, élué avec un mélange de dichlorométhane et d'éther ethylique (90-10). Après évaporation des solvants on recueille 1,4 g (73%) de l'amide attendu sous forme d'une huile.

### EXEMPLE 45

### 4-(3-tert-butyl-4-méthoxybenzoyloxyméthyl)benzaldéhyde :

a) 4-(1,3-dioxolane)benzoate de méthyle :
   Dans un ballon, on introduit 8,2 g (0,05 mole) de 4-formyl benzoate de méthyle, 25 ml d'éthylèneglycol, 3,5 g de ZnCl₂ et 200 ml de xylène. On chauffe à reflux et décante l'eau formée évapore à sec, verse le milieu réactionnel dans l'eau, extrait avec de l'éther éthylique, décante la phase organique, sèche sur sulfate de magnésium, évapore. On recueille 10,4 g (100%) du produit attendu sous forme d'une huile incolore.
b) 4-(1,3-dioxolane)benzèneméthanol :
   Dans un tricol, on introduit 1,9 g (0,05 mole) d'hydrure double de lithium et d'aluminium et 100 ml de THF. On ajoute goutte à goutte sous azote une solution de 10,4 g (0,05 mole) de l'ester méthylique précédent dissous dans 100 ml de THF et chauffe à reflux durant 4 h. On hydrolyse par addition de 3,6 ml d'une solution aqueuse de tartrate double de sodium et de potassium, filtre le sel et évapore le filtrat. On recueille 8 g (90%) de l'alcool attendu sous forme d'une huile incolore.
c) 4-hydroxyméthylbenzaldéhyde :
   Dans un ballon, on introduit 7,9 g (44 mmoles) de l'alcool précédent et 100 ml de THF, on ajoute 20 ml d'acide chlorhydrique (1N) et chauffe à reflux 4 h. On évapore à sec le milieu réactionnel, reprend par l'éther éthylique et une solution saturée de bicarbonate de sodium, décante la phase organique, sèche sur sulfate de magnésium, évapore.
   Le résidu obtenu est purifié par chromatographie sur silice élué avec du dichlorométhane. On recueille après évaporation des solvants 4,9 g (83%) du produit attendu sous forme d'une huile.
d) 4-(3-tert-butyl-4-méthoxybenzoyloxyméthyl)benzaldéhyde :
   De manière analogue à l'exemple 1(d) par réaction de 7,6 g (34 mmoles) de chlorure de 3-tert-butyl-4-méthoxybenzoyle avec 4,6 g (34 mmoles) de 4-hydroxyméthylbenzaldéhyde, on obtient 5,6 g (51%) de l'aldéhyde attendu de point de fusion 75-6°C.

### EXEMPLE 46

### 4-(3-tert-butyl-4-méthoxybenzoyloxyméthyl)benzèneméthanol :

Dans un ballon, on introduit 1,6 g (5 mmoles) de l'aldéhyde obtenu à l'exemple 45, 50 ml de THF et 50 ml de méthanol. On ajoute par petites quantités 100 mg (2,5 mmoles) de borohydrure de sodium et agite à température ambiante 4 h. On verse le milieu réactionnel dans l'eau, extrait avec de l'éther éthylique, décante la phase organique, sèche sur sulfate de magnésium, évapore.

Le résidu obtenu est trituré dans de l'hexane et de l'éther (80-20), filtré séché. On recueille 1,3 g (81%) de l'alcool attendu de point de fusion 84-5°C.

### EXEMPLE 47

### Acétate de 4-(3-tert-butyl-4-méthoxybenzoyloxyméthyl)benzèneméthanol :

Dans un ballon, on introduit 650 mg (2 mmoles) de l'alcool obtenu à l'exemple 49, 320 µl (4 mmoles) de pyridine et 50 ml de THF. On ajoute goutte à goutte une solution de 280 µl (4 mmoles) de chlorure d'acétyle dans 20 ml THF et agite à température ambiante 5 h. On verse le milieu réactionnel dans l'eau, extrait avec de l'éther éthylique, décante la phase organique, sèche sur sulfate de magnésium, évapore. Le résidu obtenu est purifié par chromatographie sur colonne de silice élué avec un mélange d'éther éthylique et d'hexane (30-70). On recueille, après évaporation des solvants 600 mg (81%) d'acétate attendu sous forme d'une huile incolore.

### EXAMPLE 48

### 4-(3-tert-butyl-4-méthoxybenzoyloxyméthyl)phénol :

Dans un ballon, on introduit 1,24 g (0,01 mole) de 4-hydroxybenzèneméthanol, 1 ml (0,01 mole) de pyridine et 50 ml de THF. En refroidissant avec un bain de glace, on introduit goutte à goutte une solution de 2,2 g (0,01 mole) de chlorure de 3-tert-butyl-4-méthoxybenzoyle dans 50 ml de THF et agite à température ambiante 24 h. On verse le milieu réactionnel dans l'eau, extrait avec de l'éther éthylique, décante la phase orcanique, sèche sur sulfate de magnésium et évapore. Le résidu obtenu est purifié par chromatographie sur colonne de silice élué avec un mélange de dichlorométhane et d'éther éthylique (97-3).

Après évaporation des solvants, on recueille 1,8 g (58%) de phénol qui fond à 112-3°C.

### EXEMPLE 49

### Acide 4-(3-tert-butyl-4-hydroxybenzoyloxyméthyl)benzoïque :

a) 4-(3-tert-butyl-4-tert-butyl-diméthylsilyloxybenzoyloxyméthyl)benzoate d'allyle :
   De manière analogue à l'exemple 1(d) par réaction de 4,9 g (15 mmoles) de chlorure de 3-tert-butyl-4-tert-butyl-diméthylsilyloxybenzoyle avec 2,9 g (15 mmoles) de 4-hydroxyméthylbenzoate d'allyle, on obtient 4,1 g (60%) de l'ester allylique sous forme d'une huile légèrement jaune.
b) Acide 4-(3-tert-butyl-4-tert-butyl-diméthylsilyloxybenzoyloxyméthyl)benzoïque :
   De manière analogue à l'exemple 3(c) à partir de 4 g (8,8 mmoles) de l'ester allylique précédent, on obtient 1,5 g (41%) d'acide de point de fusion 152-3°C.
c) Acide 4-(3-tert-butyl-4-hydroxybenzoyloxyméthyl)benzoïque :
   Dans un ballon, on introduit 1,5 g (3,6 mmoles) de l'acide précédent et 75 ml de THF. Sous courant d'azote on ajoute goutte à goutte 1,1 ml d'une solution de tétrabutylammoniumfluorure dans le THF (1 M) et agite à température ambiante pendant 1 h.
   On évapore à sec le milieu réactionnel, reprend par l'eau et l'éther éthylique, décante la phase organique, sèche sur sulfate de magnésium et évapore.
   Le résidu obtenu est trituré dans l'hexane, filtré, séché, on recueille 1 g (84%) d'acide attendu de point de fusion 221-2°C.

### EXEMPLE 50

### Acide 5-(3-tert-butyl-4-méthoxybenzoyloxyméthyl)-2-thiophènecarboxylique :

a) 5-(3-tert-butyl-4-méthoxybenzoyloxyméthyl)-2-thiophènecarboxylate d'allyle :
   De manière analogue à l'exemple 1(d) par réaction de 1,6 g (7 mmoles) de chlorure de 3-tert-butyl-4-méthoxybenzoyle avec 1,4 g (7 mmoles) de 5-hydroxyméthyl-2-thiophènecarboxylate d'allyle, on obtient 1,5 g (55%) d'ester allylique sous forme d'une huile légèrement jaune.
b) Acide 5-(3-tert-butyl-4-méthoxybenzoyloxyméthyl)-2-thiophènecarboxylique :
   De manière analogue à l'exemple 3(c) à partir de 1,5 g (4 mmoles) de l'ester allylique précédent, on obtient 930 mg (69%) de l'acide attendu de point de fusion 187-8°C.

### EXEMPLE 51

### Acide 4-(3-tert-butyl-4-isopropyloxybenzoyloxyméthyl)benzoïque :

a) 3-tert-butyl-4-isopropyloxybenzoate de benzyle :
   Dans un tricol, on introduit sous azote 5,7 g (0,02 mole) de 3-tert-butyl-4-hydroxybenzoate de benzyle, 7 g (0,05 mole) de carbonate de potassium finement broyé 4 ml (0,04 mole) de 2-iodopropane et 100 ml de méthyléthylcétone. On chauffe à reflux pendant 24 h, filtre le minéral, évapore. On reprend le résidu par l'éther éthylique et l'eau, décante la phase organique, sèche sur sulfate de magnésium, évapore.
   Le résidu obtenu est purifié par chromatographie sur silice élué avec du dichlorométhane, on recueille 6,1 g (94%) de l'ester benzylique attendu sous forme d'une huile jaune.
b) Acide 3-tert-butyl-4-isopropyloxybenzoïque :
   De manière analogue à l'exemple 2(d) à partir de 6,1 g (19 mmoles) de l'ester benzylique précédent, on obtient 3,8 g (86%) de l'acide attendu qui fond à 178-9°C.
c) Chlorure de 3-tert-butyl-4-isopropyloxybenzoyle :
   De manière analogue à l'exemple 2(b) à partir de 3,8 g (16 mmole) d'acide 3-tert-butyl-4-isopropyloxybenzoïque, on obtient 4,1 g (100%) de chlorure d'acide brut, qui est utilisé tel quel pour la suite de la synthèse.
d) 4-(3-tert-butyl-4-isopropyloxybenzoyloxyméthyl)benzoate d'allyle :
   De manière analogue à l'exemple 1(d) par réaction de 4,1 g (16 mmoles) de chlorure de 3-tert-butyl-4-isopropyloxybenzoyle avec 3,1 g (16 mmoles) de 4-hydroxyméthylbenzoate d'allyle, on obtient 5,1 g (77%) d'ester allylique de point de fusion 63-4°C.
e) Acide 4-(3-tert-butyl-4-isopropyloxybenzoyloxyméthyl)benzoïque :
   De manière analogue à l'exemple 3(c) à partir de 5,1 g (12 mmoles) de l'ester allylique précédent, on obtient 2,4 g (52%) d'acide attendu de point de fusion 186-7°C.

### EXEMPLE 52

### 4-(3,5-di-tert-butyl-4-hydroxybenzoyloxyméthyl)benzoate d'allyle :

De manière analogue à l'exemple 1(d), par réaction de 7,7 g (28,6 mmoles) de chlorure de 3,5-di-tert-butyl-4-hydroxybenzoyle avec 5,5 g (28,6 mmoles) de 4-hydroxyméthylbenzoate d'allyle on obtient 4,1 g (34%) d'ester allylique attendu de point de fusion 100-1°C.

### EXEMPLE 53

### Acide 2-hydroxy-4-(3-tert-butyl-4-méthoxybenzoyloxyméthyl)benzoïque :

a) 4-hydroxyméthyl-2-hydroxybenzoate de méthyl :
   De manière analogue à l'exemple 5(b) à partir de 3,6 g (0,02 mole) de 4-formyl-2-hydroxybenzoate de méthyle, on obtient 3,7 g (100%) de l'alcool attendu de point de fusion 77-8°C.
b) Acide 4-hydroxyméthyl-2-hydroxybenzoïque :
   De manière analogue à l'exemple 12(b) à partir de 3,7 g (0,02 mole) de 4-hydroxyméthyl-2-hydroxybenzoate de méthyle on obtient 3 g (80%) de l'acide attendu de point de fusion 179-80°C.
c) 4-hydroxyméthyl-2-hydroxybenzoate d'allyle :
   De manière analogue à l'exemple 7(a) à partir de 3 g (18 mmoles) de l'acide précédent, on obtient 3 g (82%) d'ester allylique de point de fusion 59-60°C.
d) 2-hydroxy-4-(3-tert-butyl-4-méthoxybenzoyloxyméthyl)benzoate d'allyle
   De manière analogue à l'exemple 1(d) par réaction de 2,4 g (10,5 mmoles) de chlorure de 3-tert-butyl-4-méthoxybenzoyle avec 2,2 g (10,5 mmoles) de 4-hydroxyméthyl-2-hydroxybenzoate d'allyle, on obtient 2,5 g (61%) d'ester allylique attendu sous forme d'une huile légèrement jaune.
e) Acide 2-hydroxy-4-(3-tert-butyl-4-méthoxybenzoyloxyméthyl)benzoïque :
   De manière analogue à l'exemple 3(c) à partir de 2,3 g (6mmoles) de l'ester allylique précédent, on obtient 595 mg (28%) d'acide attendu de point de fusion 150-1°C.

### EXEMPLE 54

### N-morpholinyl-2-hydroxy-4-(5,6,7,8-tétrahydro-5,5,8,8-tétraméthyl-2-naphtoylméthyloxy)benzamide :

a) Chlorure de 2-hydroxy-4-(5,6,7,8-tétrahydro-5,5,8,8-tétraméthyl-2-naphtoylméthyloxy)benzoyle :
   A une suspension de 25,5 g (67 mmoles) de l'acide obtenu à l'exemple 32, dans 255 ml de toluène et 2,55 ml de DMF chauffée à 80°C, on ajoute goutte à goutte 5,36 ml (73,7 mmoles) de chlorure de thionyle.
   On agite 1 h à 80°C, puis le solvant est évaporé sous vide. Le chlorure d'acide obtenu (26,7 g) mis en solution dans le THF est utilisé directement pour la préparation des amides.
b) N-morpholinyl-2-hydroxy-4-(5,6,7,8-tétrahydro-5,5,8,8-tétraméthyl-2-naphtoylméthyloxy)benzamide :
   Une solution de 8,9 g (22 mmoles) du chlorure d'acide précédent, dans 90 ml de THF est ajoutée goutte à goutte à un mélange de 17,5 g (200 mmoles) de morpholine et 90 ml de THF à température ambiante. On agite encore 1 h puis verse sur de l'eau et acidifie à pH 5-6 par de l'HCl.
   On extrait à l'acétate d'éthyle, lave à l'eau et sèche la phase organique sur sulfate de sodium. Par évaporation on obtient 12 g d'amide brut, qui est purifié par chromatographie sur colonne de silice élué avec un mélange d'acétate d'éthyle et d'hexane (45-55). On recueille 8,55 g (85,5%) d'un produit blanc de point de fusion 166-7°C.

### EXEMPLE 55

### 2-hydroxy-4-(5,6,7,8-tétrahydro-5,5,8,8-tétraméthyl-2-naphtoylméthyloxy)benzamide :

Une solution de 8,9 g (22 mmoles) du chlorure d'acide décrit à l'exemple 54(a), dans 90 ml de THF est ajoutée goutte à goutte à 100 ml d'une solution saturée d'ammoniac dans le THF à température ambiante. On agite encore 1 h puis verse sur de l'eau et acidifie à pH 5-6 par de l'HCl.

On extrait à l'acétate d'éthyle, lave à l'eau et sèche la phase organique sur sulfate de sodium. Le produit brut obtenu par évaporation est chromatographié sur colonne de silice élué avec un mélange d'acétate d'éthyle et d'hexane (40-60). On recueille 6,94 g (82,6%) de poudre blanc-crème de point de fusion 196-7°C.

### EXEMPLE 56

### N-Piperidinyl-2-hydroxy-4-(5,6,7,8-tétrahydro-5,5,8,8-tétraméthyl-2-naphtoylméthyloxy)benzamide :

Une solution de 6 g (15 mmoles) du chlorure d'acide décrit à l'exemple 54 (a), dans 60 ml de THF est ajoutée goutte à goutte à un mélange de 4,44 ml (45 mmoles) de piperidine et 60 ml de THF à température ambiante. On agite encore 2 h puis verse sur de l'eau et acidifie à pH 6-7 par de l'HCl.

On extrait à l'acétate d'éthyle, lave à l'eau et sèche la phase organique sur sulfate de sodium. Le produit brut obtenu (7 g) est cristallisé avec un mélange d'acétate d'éthyle et d'hexane (30-70). On recueille 5,04 g (75,2%) de poudre blanc-crème de point de fusion 120-4°C.

### EXEMPLE 57

### N-éthyl-2-hydroxy-4-(5,6,7,8-tétrahydro-5,5,8,8-tétraméthyl-2-naphtoylméthyloxy)benzamide :

Une solution de 8,9 g (22 mmoles) du chlorure d'acide décrit à l'exemple 54(a), dans 90 ml de THF est ajoutée goutte à goutte à 67 ml d'une solution 3 N d'éthylamine dans l'éthanol à température ambiante. On agite 2 h puis verse sur de l'eau et extrait à l'acétate d'éthyle après avoir acidifié par de l'HCl jusqu'à pH 5-6.

La phase organique est lavée à l'eau puis séchée sur sulfate de sodium. Le produit brut obtenu par évaporation sous vide est chromatographié sur colonne de silice élué avec un mélange d'acétate d'éthyle et d'hexane (20-80). On recueille 970 mg d'une poudre jaune pâle de point de fusion 134°C.

### EXEMPLE 58

### Acide 2-hydroxy-4-[3-(1-adamantyl)-4-méthoxybenzoylméthyloxy]benzoïque :

a) 3-(1-adamantyl)-4-méthoxyphényléthanone :
   15,75 g (0,055 mole) d'acide 3-(1-adamantyl)-4-méthoxybenzoïque en solution dans 150 ml de toluène sont traités par 7,3 ml de chlorure de thionyle et sont chauffés à 100°C durant 3 h 30. Le milieu réactionnel est évaporé à sec, puis on ajoute sous azote à ce résidu d'évaporation 30 ml d'hexaméthyl-phosphoramide, 8 ml (0,0575 mole) de tétraméthylétain et 22 mg de chlorure de benzylbis(triphenylphosphine)palladium(II). Le milieu réactionnel est chauffé à 65°C pendant 30 mn puis est laissé sous agitation à température ambiante durant la nuit. Le milieu réactionnel est versé dans l'eau et extrait à l'éther. On isole après chromatographie sur silice dans l'éluant dichlorométhane/hexane (60-40) 10,47 g (71%) du dérivé attendu fondant à 138-140°C.
b) [3-(1-adamantyl)-4-méthoxyphényl]-2-bromoéthanone :
   5,63 g (19,8 mmoles) de la cétone obtenue dans l'exemple 58(a), en solution dans 30 ml de dioxanne et 30 ml d'éther éthylique, sont traités par 1 ml de brome (19,8 mmoles) en solution dans 15 ml de dichlorométhane. Le milieu réactionnel est laissé sous agitation 30 mn à température ambiante puis est versé dans 200 ml d'eau et extrait par 700 ml d'éther éthylique. Après lavage et séchage de la phase organique, on isole 6,7 g (93%) du dérivé attendu fondant à 140-1°C.
c) 2-hydroxy-4-[3-(1-adamantyl)-4-méthoxybenzoylméthyloxy]benzoate de benzyle:
   4,90 g (20 mmoles) de 2,4-dihydroxybenzoate de benzyle sont traités par 605 mg d'hydrure de sodium (80% dans l'huile) dans 50 ml de DMF. On ajoute alors goutte à goutte 7,29 g (20 mmoles) du dérivé bromé obtenu à l'exemple précédent en solution dans 100 ml de DMF et laisse réagir sous agitation 2 h à température ambiante. On isole après traitement habituel 7,39 g (70%) du dérivé attendu fondant à 129°C.
d) Acide -2-hydroxy-4-[3-(1-adamantyl)-4-méthoxybenzoylméthyloxy]benzoïque :
   3,68 g (6,98 mmoles) de l'ester obtenu à l'exemple 58(c), dans 50 ml de dioxanne et 0,5 ml d'acide acétique, sont hydrogénés en présence de 57 mg de palladium sur charbon (10%) à 40°C sous une pression de 7 bars d'hydrogène durant 3 h 30. On isole après traitement et recristallisation dans le mélange eau/éthanol 2,32 g (76%) du dérivé attendu fondant à 225-6°C.

### EXEMPLE 59

### (-) Acide 4-[[1-(5,6,7,8-tétrahydro-5,5,8,8-tétraméthyl-2-naphtyl)éthyloxyl carbonyl]benzoïque :

a) (+) 1-(5,6,7,8-tétrahydro-5,5,8,8-tétraméthyl-2-naphtyl)éthanol :
   6 g de (±) acétoxy-1-(5,6,7,8-tétrahydro-5,5,8,8-tétraméthyl-2-naphtyl)éthane en suspension dans un mélange de 100 ml tampon phosphate (0,3 M, pH 7) et 1 ml de chloroforme sont traités par 0,87 g de lipase amano P30 sous agitation à 40°C pendant 3 jours. On isole 2,06 g (88%) du produit attendu fondant à 61-2°C (α_{D} = +27° : c=1, éthanol)) et 2,2 g (73%) d'acétoxy-1-(5,6,7,8-tétrahydro-5,5,8,8-tétraméthyl-2-naphtyl)éthane.
b) (-) 4-[[1-(5,6,7,8-tétrahydro-5,5,8,8-tétraméthyl-2-naphtyl)éthloxy]carbonyl] benzoate d'allyle :
   A une solution de 1,73 g (7,44 mmoles) de l'alcool (+) 1-(5,6,7,8-tétrahydro-5,5,8,8-tétraméthyl-2-naphtyl)éthylique et de 1,54 g (7,44 mmoles) de monotéréphtalate d'allyle dans 50 ml de THF, on ajoute 1,54 g (7,44 mmoles) de dicyclohexylcarbodiimide et 0,91 g de 4-diméthylaminopyridine. Le milieu réactionnel est laissé sous agitation à température ambiante pendant 3 h. Après traitement et chromatographie sur silice dans le dichlorométhane on isole 2,42 g (77%) du dérivé attendu sous forme d'huile incolore (Rf = 0,46, dichlorométhane/hexane 50-50).
c) (-) Acide 4-[[1-(5,6,7,8-tétrahydro-5,5,8,8-tétraméthyl-2-naphtyl)éthyloxy] carbonyl]benzoïque :
   Une solution de 2,40 g (5,71 mmoles) de l'ester obtenu à l'exemple 59(b) dans 30 ml de THF est traitée par 340 mg de tétrakis(triphénylphosphine)palladium(0) et 5 ml de morpholine dans les conditions de préparation de l'exemple 24(b) pour conduire à 1,94 g (89%) du dérivé attendu fondant à 138°C (α_{D} = -53,2° ; c=1, éthanol)

### EXEMPLE 60

### (+) Acide 4-[[1-(5,6,7,8-tétrahydro-5,5,8,8-tétraméthyl-2-naphtyl)éthyloxy] carbonyl]benzoïque :

a) (-) 1-(5,6,7,8-tétrahydro-5,5,8,8-tétraméthyl-2-naphtyl)éthanol :
   2,2 g (8 mmoles) obtenus à l'exemple 59(a) d'acétoxy-1-(5,6,7,8-tétrahydro-5,5,8,8-tétraméthyl-2-naphtyl)éthane en solution dans 20 ml de méthanol sont traités par 1,6 g de soude et laissés sous agitation durant 24 h à température ambiante. Après évaporation et acidification par HCl 1 N, le précipité est extrait par de l'éther éthylique pour conduire à 1,75 g (93%) de l'alcool attendu fondant à 61-2°C (α_{D} = -27,2°; c=1, éthanol)
b) (+) 4-[[1-(5,6,7,8-tétrahydro-5,5,8,8-tétraméthyl-2-naphtyl)éthyloxy] carbonyl]benzoate d'allyle :
   1,69 g (7,27 mmoles) de l'alcool obtenu à l'exemple 60(a) est condensé avec 1,5 g de monotéréphtalate d'allyle dans les conditions décrites à l'exemple 22(a) pour conduire après traitement et chromatographie sur silice à 2,54 g (83%) de l'ester attendu sous forme d'huile incolore. Rf. 0,46 (dichlorométhane/hexane 50-50)
c) (+) Acide 4-[[1-(5,6,7,8-tétrahydro-5,5,8,8-tétraméthyl-2-naphtyl)éthyloxy] carbonyl]benzoïque :
   2,53 g (6 mmoles) d'ester obtenu à l'exemple 60(b) sont traités dans les conditions décrites pour la préparation de l'exemple 22(b) pour conduire à 1,72 g (75%) du dérivé attendu fondant à 138°C. (α_{D} = +52,1 ; c=1, éthanol).

### EXEMPLES DE FORMULATIONS

### A. VOIE ORALE

(a) Comprimé dé 0,2 g

| | |
|---|---|
| Acide 4-(5,6,7,8-tétrahydro-5,5,8,8-tétraméthyl -2-naphtylméthyloxycarbonyl)benzoïque | 0,001 g |
| Amidon | 0,114 g |
| Phosphate bicalcique | 0,020 g |
| Silice | 0,020 g |
| Lactose | 0,030 g |
| Talc | 0,010 g |
| Stéarate de magnésium | 0,005 g |

Dans cet exemple l'acide 4-(5,6,7,8-tétrahydro-5,5,8,8-tétraméthyl -2-naphtylméthyloxycarbonyl)benzoïque peut être remplacé par l'acide 4-(5,6,7,8-tétrahydro-5,5,8,8-tétraméthyl-2-naphtoyloxyméthyl) benzoïque.
(b) Suspension buvable en ampoules de 5 ml

| | |
|---|---|
| Acide 4-(5,6,7,8-tétrahydro-5,5,8,8-tétraméthyl -2-naphtyloxycarboxamido)benzoïque | 0,001 g |
| Glycérine | 0,500 g |
| Sorbitol à 70 % | 0,500 g |
| Saccharinate de sodium | 0,010 g |
| Parahydroxybenzoate de méthyle | 0,040 g |
| Arôme qs | |
| Eau purifiée qsp | 5 ml |

Dans cet exemple, l'acide 4-(5,6,7,8-tétrahydro-5,5,8,8-tétraméthyl -2-naphtyloxycarboxamido)benzoïque peut être remplacé par l'acide 4-(5,6,7,8-tétrahydro-5,5,8,8-tétraméthyl-2-naphtylacétamido)benzoïque
(c) Comprimé de 0,8 g

| | |
|---|---|
| Acide 4-[1-(5,6,7,8-tétrahydro-5,5,8,8-tétraméthyl -2-naphtoyloxy)éthyl]benzoïque | 0,500 g |
| Amidon prégélatinisé | 0,100 g |
| Cellulose microcristalline | 0,115 g |
| Lactose | 0,075 g |
| Stéarate de magnésium | 0,010 g |

Dans cet exemple, l'acide 4-[1-(5,6,7,8-tétrahydro-5,5,8,8-tétraméthyl -2-naphtoyloxy)éthyl]benzoïque peut-être remplacé par l'acide 4-(5,6,7,8-tétrahydro-5,5,8,8-tétraméthyl-2-naphtoylméthyloxy)benzoïque
(d) Suspension buvable en ampoules de 10 ml

| | |
|---|---|
| Acide 4-(5,6,7,8-tétrahydro-5,5,8,8-tétraméthyl-2-naphtoyl formamido)benzoïque | 0,05 g |
| Glycérine | 1,000 g |
| Sorbitol - 70 % | 1,000 g |
| Saccharinate de sodium | 0,010 g |
| Para-hydroxybenzoate de méthyle | 0,080 g |
| Arôme qs | |
| Eau purifiée qsp | 10 ml |

Dans cet exemple l'acide 4-(5,6,7,8-tétrahydro-5,5,8,8 tétraméthyl-2-naphtoylformamido)benzoïque peut être remplacé par l'acide 4-(α-hydroxy-5,6,7,8-tétrahydro-5,5,8,8-tétraméthyl-2-naphtylacétamido)benzoïque ou par l'acide 2-hydroxy-4-(5,6,7,8-tétrahydro-5,5,8,8-tétraméthyl-2-naphtoyl-méthyloxy)benzoïque.

### B. VOIE TOPIQUE

(a) Onguent

| | |
|---|---|
| Acide 4-(5,6,7,8-tétrahydro-5,5,8,8-tétraméthyl -2-naphtylacétoxy)benzoïque | 0,020 g |
| Myristate d'isopropyle | 81,700 g |
| Huile de vaseline fluide | 9,100 g |
| Silice vendue par la Société DEGUSSA sous la dénomination "Aérosil 200" | 9,180 g |

Dans cet exemple, l'acide 4-(5,6,7,8-tétrahydro-5,5,8,8-tétraméthyl -2-naphtylacétoxy)benzoïque peut-être remplacé par l'acide 4-(5,6,7,8-tétrahdyro-5,5,8,8-tétraméthyl-2-naphtyloxycarboxamido) benzoïque.
(b) Onguent

| | |
|---|---|
| Acide 4-(5,6,7,8-tétrahydro-5,5,8,8-tétraméthyl -2-naphtoylthiométhyl)benzoïque | 0,300 g |
| Vaseline blanche codex qsp | 100 g |

Dans cet exemple l'acide 4-(5,6,7,8-tétrahydro-5,5,8,8-tétraméthyl -2-naphtoylthiométhyl)benzoïque peut être remplacé par l'acide 4-(5,6,7,8-tétrahydro-5,5,8,8-tétraméthyl-2-naphtylacétamido)benzoïque.
(c) Crème Eau-dans-l'Huile non ionique

| | |
|---|---|
| Acide 4-(α-méthylène-5,6,7,8-tétrahdyro-5,5,8,8 -tétraméthyl-2-naphtylacétoxy)benzoïque | 0,100 g |
| Mélange d'alcools de lanoline émulsifs, de cires et d'huiles raffinés, vendu par la Société BDF sous la dénomination "Eucerine anhydre" | 39,900 g |
| Parahydroxybenzoate de méthyle | 0,075 g |
| Parahydroxybenzoate de propyle | 0,075 g |
| Eau déminéralisée stérile qsp | 100 g |

Dans cet exemple, l'acide 4-(α-méthylène-5,6,7,8-tétrahydro-5,5,8,8 -tétraméthyl-2-naphtylacétoxy)benzoïque peut-être remplacé par l'acide 4-[1-(5,6,7,8-tétrahydro-5,5,8,8-tétraméthyl-2-naphtoyloxy)éthyl]benzoïque.
(d) Lotion

| | |
|---|---|
| Acide 4-(3-tert-butyl-4-méthoxybenzoyloxyméthyl)benzoïque | 0,100 g |
| Polyéthylène glycol (PEG 400) | 69,900 g |
| Ethanol 95 % | 30,000 g |

Dans cet exemple, l'acide 4-(5,6,7,8-tétrahydro-5,5,8,8-tétraméthyl-2-naphtyloxyacétyl) benzoïque peut être remplacé par l'acide 4-(5,6,7,8-tétrahydro-5,5,8,8-tétraméthyl-2-naphtylméthyloxycarbonyl) benzoïque.
(e) Onguent hydrophobe

| | |
|---|---|
| Acide 4-(5,6,7,8-tétrahydro-5,5,8,8-tétraméthyl-2-naphtoyloxyméthyl) benzoïque | 0,300 g |
| Myristate d'isopropyle | 36,400 g |
| Huile de silicone vendue par la Société Rhône Poulenc sous la dénomination "Rhodorsil 47 V 300" | 36,400 g |
| Cire d'abeille | 13,600 g |
| Huile de silicone vendue par la Société Goldschmidt sous la dénomination "Abil 300.000 cst" qsp | 100 g |

Dans cet exemple, l'acide 4-(5,6,7,8-tétrahydro-5,5,8,8-tétraméthyl-2-naphtoyloxyméthyl) benzoïque peut être remplacé par l'acide 4-(3,5-di-tert-butyl-4-hydroxybenzoyloxyméthyl) benzoïque.
(f) Crème Huile-dans-l'Eau non ionique

| | |
|---|---|
| Acide 5-(5,6,7,8)-tétrahydro-5,5,8,8-tétraméthyl-2-naphtoyloxyméthyl)-2 thiophènecarboxylique | 0,500 g |
| Alcool cétylique | 4,000 g |
| Monostéarate de glycérol | 2,500 g |
| Stéarate de PEG 50 | 2,500 g |
| Beurre de karité | 9,200 g |
| Propylène glycol | 2,000 g |
| Parahydroxybenzoate de méthyle | 0,075 g |
| Parahydroxybenzoate de propyle | 0,075 g |
| Eau déminéralisée stérile qsp | 100 g |

## Revendications

1. Composés bi-aromatiques, caractérisés par le fait qu'ils répondent à la formule générale suivante : 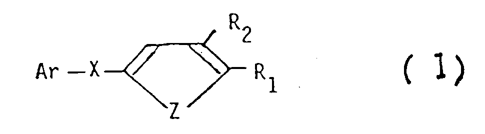 dans laquelle :
Ar représente soit le radical 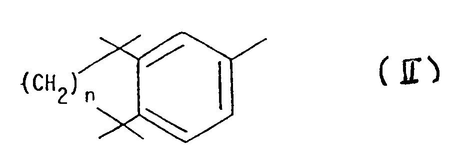 avec n = 1 ou 2
soit le radical 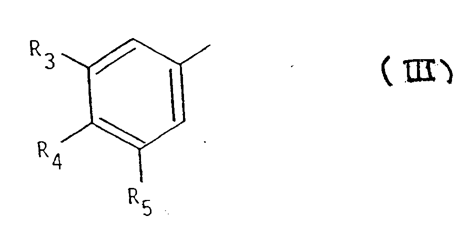
R₃ et R₅ représentant un atome d'hydrogène, le groupe OH, un radical alcoxy ayant de 1 à 6 atomes de carbone, un radical alkyle α-substitué ayant de 3 à 12 atomes de carbone ou un radical alkyle α,α'-disubstitué ayant de 4 à 12 atomes de carbone, un radical cycloalkyle ayant de 3 à 12 atomes de carbone, un radical cycloalkyle mono ou polycyclique ayant de 5 à 12 atomes de carbone dont le carbone de liaison est trisubstitué,
R₄ représentant un atome d'hydrogène, le groupe OH, un radical alcoxy ayant de 1 à 6 atomes de carbone, un radical alkyle α-substitué ayant de 3 à 12 atomes de carbone, un radical alkyle α,α'-disubstitué ayant de 4 à 12 atomes de carbone, un radical cycloalkyle ayant de 3 à 12 atomes de carbone, un radical cyclcoalkyle mono ou polycyclique ayant de 5 à 12 atomes de carbone dont le carbone de liaison est trisubstitué, un radical monohydroxyalkyle, un radical polyhydroxyalkyle, un atome de fluor, un atome de chlore, le groupe SH, le groupe SR₆, le groupe SOR₆, le groupe SO₂R₆, un radical alkényle ayant de 2 à 6 atomes de carbone, ou un radical alkényloxy ayant de 2 à 6 atomes de carbone,
R₆ représentant un radical alkyle inférieur,
R₁ représente un atome d'hydrogène, le groupe OH, le radical -CH₃, le radical -CH₂OH, le radical -COR₇,, le radical -CH(OH)CH₃, le radical -CH₂OCOR₈, le radical -SO₂R₉, le radical -SOR₉ ou le radical -SR₉,
R₇ représentant un atome d'hydrogène, le groupe OH, le radical -OR₁₀, le radical -N(r'r''), un radical alkyle inférieur, un radical monohydroxyalkyle, un radical polyhydroxyalkyle ou le reste d'un sucre,
R₁₀ représentant un radical alkyle ayant de 1 à 12 atomes de carbone ou un radical alkényle ayant de 2 à 12 atomes de carbone,
r' et r'' identiques ou différents, représentent un atome d'hydrogène, un radical alkyle inférieur, un radical aryle, un radical aralkyle, un reste d'aminoacide, un reste de sucre, un reste de sucre aminé ou un hétérocycle, ou r' et r'' pris ensemble forment un hétérocycle,
R₈ représentant un radical alkyle linéaire ou ramifié saturé ou insaturé ayant de 1 à 20 atomes de carbone ou le reste d'un sucre,
R₉ représentent le groupe OH, un radical alkyle inférieur ou le radical -N(r'r''),
R₂ représentant un atome d'hydrogène, le groupe OH, un radical alkyle inférieur, un radical alcoxy ayant de 1 à 6 atomes de carbone, un atome de fluor, un atome de chlore, le groupe CF₃, le groupe COR₇, le groupe CH₂OH ou le groupe CH₂OR₆, Z représente un atome d'oxygène ou de soufre, le radical divalent -CH=CR₁₁-, le radical divalent -N=CH- ou le radical divalent -N=CR₆-,
R₁₁ représentant un atome d'hydrogène, le groupe OH, le radical alkyle inférieur, un radical alcoxy ayant de 1 à 6 atomes de carbone, un atome de fluor, un atome de chlore, ou le groupe CF₃,
X est un radical divalent qui peut être lu de gauche à droite ou inversement, choisi dans le groupe constitué par :
(i) 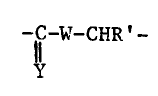 R' représentant un atome d'hydrogène, le radical -CH₃
W représentant un atome d'oxygène ou de soufre ou le groupe -NR',
Y représentant un atome d'oxygène ou bien un atome de soufre lorsque W représente le groupe -NR',
(ii) 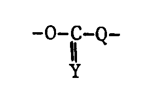 Q représentant un atome d'oxygène ou -NR',
Y représentant un atome d'oxygène ou bien un atome de soufre lorsque Q représente le groupe -NR',
(iii) 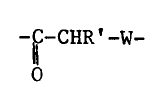
(iv) 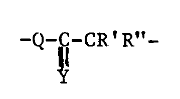 R'' représentant un atome d'hydrogène, le radical -CH₃, le groupe OH, un atome de fluor ou un atome de chlore, ou
R' et R'' pris ensemble forment un radical méthano (=CH₂) ou un radical oxo (=O),
(v) 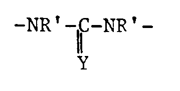 Y représentant un atome d'oxygène ou un atome de soufre,
sous réserve que lorsque simultanément X représente 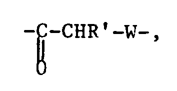 Z représente -CH = CH- et R₂ représente un atome d'hydrogène alors Ar représente le radical de formule (III) dans laquelle R₃ et R₅ sont différents d'un atome d'hydrogène ou d'un radical alkyle α-substitué ou α-αdisubstitué ayant de 3 à 5 atomes de carbone,
et les sels des composés de formule (I) lorsque R₁ représente une fonction acide carboxylique et les isomères optiques desdits composés de formule (I).

2. Composés selon la revendication 1, caractérisés par le fait qu'ils se présentent sous forme de sels d'un métal alcalin ou alcalino-terreux ou encore de zinc ou d'une amine organique.

3. Composés selon la revendication 1, caractérisés par le fait que le radical alkyle inférieur a de 1 à 6 atomes de carbone et est pris dans le groupe constitué par les radicaux méthyle, éthyle, isopropyle, butyle et tertiobutyle.

4. Composés selon la revendication 1, caractérisés par le fait que le radical alcoxy ayant de 1 à 6 atomes de carbones est pris dans le groupe constitué par un radical méthoxy, éthoxy, isopropoxy et butoxy.

5. Composés selon la revendication 1, caractérisés par le fait que le radical alkyle α-substitué est pris dans le groupe constitué par : un radical isopropyle, 1-méthylpropyle et 1-éthylpropyle.

6. Composés selon la revendication 1, caractérisés par le fait que le radical alkyle α,α'-disubstitué est pris dans le groupe constitué par : un radical tert-butyle, 1,1-diméthyl propyle, 1-méthyl 1-éthyl propyle, 1-méthyl 1-éthyl hexyle et 1,1-diméthyl décyle.

7. Composés selon la revendication 1, caractérisés par le fait que le radical monohydroxyalkyle est un radical 2-hydroxyéthyle, 2-hydroxypropyle ou 3-hydroxypropyle.

8. Composés selon la revendication 1, caractérisés par le fait que le radical polyhydroxyalkyle comporte de 3 à 6 atomes de carbone et de 2 à 5 groupes hydroxyles et est pris dans le groupe constitué par le radical 2,3-dihydroxypropyle, 2,3,4-trihydroxybutyle, 2,3,4,5-tétrahydroxy pentyle et le reste du pentaérythritol.

9. Composés selon la revendication 1, caractérisés par le fait que le radical aryle est un radical phényle éventuellement substitué par au moins un atome d'halogène, un hydroxyle ou une fonction nitro.

10. Composés selon la revendication 1, caractérisés par le fait que le radical aralkyle est le radical benzyle ou phénéthyle éventuellement substitué par au moins un atome d'halogène, un hydroxyle ou une fonction nitro.

11. Composés selon la revendication 1, caractérisés par le fait que le radical cycloalkyle mono ou polycyclique ayant de 5 à 12 atomes de carbone dont le carbone de liaison est trisubstitué, est le radical 1-méthyl cyclohexyle ou 1-adamantyle.

12. Composés selon la revendication 1, caractérisés par le fait que l'hétérocycle est pris dans le groupe constitué par un radical pipéridino, morpholino, pyrrolidino ou pipérazino, éventuellement substitué en position 4 par un radical alkyle en C₁-C₆ ou mono ou polyhydroxyalkyle.

13. Composés selon l'une quelconque des revendications précédentes, caractérisés par le fait qu'ils sont pris dans le groupe constitué par :
Acide 4-(5,6,7,8-tétrahydro-5,5,8,8-tétraméthyl-2-naphtylglyoxyloyloxy)benzoïque,
Acide 4-(5,6,7,8-tétrahydro-5,5,8,8-tétraméthyl-2-naphtylacétoxy) benzoïque,
Acide 4-(5,6,7,8-tétrahydro-5,5,8,8-tétraméthyl-2-naphtylacétamido) benzoïque,
Acide 4-(α-méthylène-5,6,7,8-tétrahydro-5,5,8,8-tétraméthyl-2-naphtylacétoxy)benzoïque,
Acide 4-(5,6,7,8-tétrahydro-5,5,8,8-tétaméthyl-2-naphtoyloxyméthyl)benzoïque,
Acide 4-(5,6,7,8-tétrahydro-5,5,8,8-tétraméthyl-2-naphtoylthiométhyl)benzoïque,
Acide 4-(5,6,7,8-tétrahydro-5,5,8,8-tétraméthyl-2-naphtyl carboxamido méthyl)benzoïque,
Acide 4-(N-méthyl-5,6,7,8-tétrahydro-5,5,8,8-tétraméthyl-2-naphtylcarboxamidométhyl)benzoïque,
Acide 4-(5,6,7,8-tétrahydro-5,5,8,8-tétraméthyl-2-naphtylméthyloxy carbonyl)benzoïque,
Acide 4-[5,6,7,8-tétrahydro-5,5,8,8-tétaméthyl-2-naphthyl (méthylthio)carbonyl]benzoïque,
Acide 5-(5,6,7,8-tétrahydro-5,5,8,8-tétraméthyl-2-naphtoyloxy méthyl)-2-thiophènecarboxylique,
Acide 4-(5,6,7,8-tétrahydro-5,5,8,8-tétraméthyl-2-naphtyloxy carboxamido)benzoïque
Acide 4-(5,6,7,8-tétrahydro-5,5,8,8-tétraméthyl-2-naphtyl carbonyldioxy)benzoïque,
Acide 4-[1-(5,6,7,8-tétrahydro-5,5,8,8-tétraméthyl-2-naphtoyloxy) éthyl]benzoïque,
Acide 4-[[1-(5,6,7,8-tétrahydro-5,5,8,8-tétraméthyl-2-naphtyl) éthyloxy]carbonyl]benzoïque,
Acide 4-(3,5-di-tert-butyl-4-hydroxybenzoyloxyméthyl)benzoïque,
Acide 4-[3-(1-adamantyl)-4-méthoxybenzoyloxyméthyl]benzoïque,
Acide 4-(3-tert-butyl-4-méthoxybenzoyloxyméthyl)benzoïque,
Acide 4-(4-tert-butylbenzoyloxyméthyl)benzoïque,
Acide 4-[4-(1-adamantyl)-3-méthoxybenzoyloxyméthyl)]benzoïque,
Acide 4-[3-(1-adamantyl)-4-méthoxyphénoxycarboxamido]benzoïque,
Acide 4-[3-(1-adamantyl)-4-méthoxyphénylcarbonyldioxy]benzoïque,
Acide 4-[3-(1-adamantyl)-4-méthoxyphénylacétamido]benzoïque,
Acide 4-(5,6,7,8-tétrahydro-5,5,8,8-tétraméthyl-2-naphtoyl formamido)benzoïque,
Acide 4-(α-hydroxy-5,6,7,8-tétrahydro-5,5,8,8-tétraméthyl-2-naphtyl acétamido)benzoïque,
Acide 4-(5,6,7,8-tétrahydro-5,5,8,8-tétraméthyl-2-naphtylcarbamoylméthyl) benzoïque,
Acide 2-hydroxy-4-(5,6,7,8-tétrahydro-5,5,8,8-tétraméthyl-2-naphtylacétoxy) benzoïque,
Acide 4-(5,6,7,8-tétrahydro-5,5,8,8-tétraméthyl-2-naphtyloxycarbonylméthyl) benzoïque,
Acide 4-(N-méthyl-5,6,7,8-tétrahydro-5,5,8,8-tétraméthyl-2-naphtylacétamido) benzoïque,
Acide 4-(α-fluoro-5,6,7,8-tétrahydro-5,5,8,8-tétraméthyl-2-naphtylacétamido) benzoïque,
Acide 4-[3-(1-adamantyl)-4-méthoxyphényluréido]benzoïque,
Acide 2-hydroxy-4-(5,6,7,8-tétrahydro-5,5,8,8-tétraméthyl-2-naphtoylméthyloxy)benzoïque,
Acide 4-[2-(5,6,7,8-tétrahydro-5,5,8,8-tétraméthyl-2-naphtyl)propionamido]benzoïque,

14. Composition pharmaceutique, caractérisée par le fait qu'elle contient dans un véhicule approprié, pour une administration par voie entérale, parentérale, topique ou oculaire, au moins un composé de formule (I) selon l'une quelconque des revendications 1 à 13.

15. Composition selon la revendication 14, caractérisée par le fait qu'elle contient de 0,0001 à environ 5% en poids d'un composé de formule (I)

16. Utilisation d'un composé selon l'une quelconque des revendications 1 à 13 pour la préparation d'une composition pharmaceutique destinée au traitement des affections dermatologiques, rhumatismales, respiratoires ainsi qu'ophtalmologiques.

17. Composition cosmétique pour l'hygiène corporelle et capillaire, caractérisée par le fait qu'elle contient, dans un véhicule cosmétique approprié, au moins un composé de formule (I) selon l'une quelconque des revendications 1 à 13.

18. Composition cosmétique selon la revendication 17, caractérisée par le fait qu'elle contient le composé de formule (I) à une concentration comprise entre 0,0001 et 0,1% et de préférence entre 0,001 et 0,01% en poids.

## Claims

1. Bi-aromatic compounds, characterized by the fact that they correspond to the following general formula: 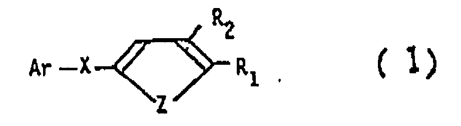 in which:
Ar represents either the radical 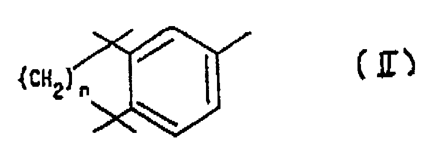 with n = 1 or 2
or the radical 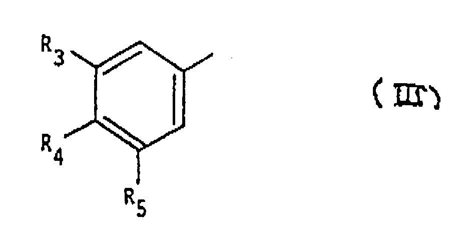
R₃ and R₅ representing a hydrogen atom, the OH group, an alkoxy radical having 1 to 6 carbon atoms, an α-substituted alkyl radical having 3 to 12 carbon atoms or an α,α'-disubstituted alkyl radical having 4 to 12 carbon atoms, a cycloalkyl radical having 3 to 12 carbon atoms, a mono- or polycyclic cycloalkyl radical having 5 to 12 carbon atoms of which the bonding carbon is trisubstituted,
R₄ represents a hydrogen atom, the OH group, an alkoxy radical having 1 to 6 carbon atoms, an α-substituted alkyl radical having 3 to 12 carbon atoms, an α,α'-disubstituted alkyl radical having 4 to 12 carbon atoms, a cycloalkyl radical having 3 to 12 carbon atoms, a mono- or polycyclic cycloalkyl radical having 5 to 12 carbon atoms of which the bonding carbon is trisubstituted, a monohydroxyalkyl radical, a polyhydroxyalkyl radical, a fluorine atom, a chlorine atom, the SH group, the SR₆ group, the SOR₆ group, the SO₂R₆ group, in alkenyl radical having 2 to 6 carbon atoms, or an alkenyloxy radical having 2 to 6 carbon atoms,
R₆ representing a lower alkyl radical,
R₁ represents a hydrogen atom, the OH group, the -CH₃ radical, the -CH₂OH radical, the -COR₇ radical, the -CH(OH)CH₃ radical, the -CH₂OCOR₈ radical, the -SO₂R₉ radical, the -SOR₉ radical or the -SR₉ radical,
R₇ representing a hydrogen atom, the OH group, the -OR₁₀ radical, the -N(r'r'') radical, a lower alkyl radical, a monohydroxyalkyl radical, a polyhydroxyalkyl radical or the residue of a sugar,
R₁₀ representing an alkyl radical having 1 to 12 carbon atoms or an alkenyl radical having 2 to 12 carbon atoms,
r' and r'', which are identical or different, represent a hydrogen atom, a lower alkyl radical, an aryl radical, an aralkyl radical, an amino acid residue, a sugar residue, on amino sugar residue or a heterocycle, where r' and r'', taken together, form a heterocycle,
R₈ representing a saturated or unsaturated linear or branched alkyl radical having 1 to 20 carbon atoms or the residue of a sugar,
R₉ representing the OH group, a lower alkyl radical or the -N(r'r'') radical,
R₂ representing a hydrogen atom, the OH group, a lower alkyl radical, an alkoxy radical having 1 to 6 carbon atoms, a fluorine atom, a chlorine atom, the CF₃ group, the COR₇ group, the CH₂OH group or the CH₂OR₆ group,
Z represents an oxygen or sulphur atom, the divalent radical -CH=CH₁₁-, the divalent radical -N=CH- or the divalent radical -N=CR₆-,
R₁₁ representing a hydrogen atom, the OH group, the lower alkyl radical, an alkoxy radical having 1 to 6 carbon atoms, a fluorine atom, a chlorine atom, or the group CF₃,
X is a divalent radical which can be read from the left to the right or conversely, chosen from the group consisting of:
(i) 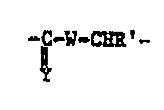 R' representing a hydrogen atom, the -CH₃ radical
W representing an oxygen or sulphur atom or the -NR' group,
Y representing an oxygen atom or alternatively a sulphur atom when W represents the -NR' group,
(ii) 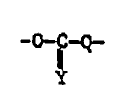 Q representing an oxygen atom or -NR',
Y representing an oxygen atom or alternatively a sulphur atom when Q represents the -NR' group,
(iii) 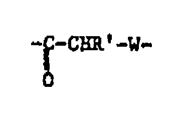
(iv) 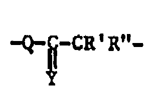 R'' representing a hydrogen atom, the -CH₃ radical, the OH group, a fluorine atom or a chlorine atom, or
R' and R'', taken together, form a methano radical (=CH₂) or an oxo radical (=O),
(v) 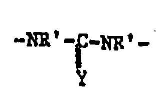 Y representing an oxygen atom or a sulphur atom, with the proviso that when simultaneously X represents 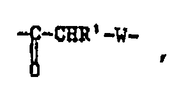 Z represents -CH=CH- and R₂ represents a hydrogen atom, then Ar represents the radical of formula (III) in which R₃ and R₅ are different from a hydrogen atom or from an α-substituted or α,α'-disubstituted alkyl radical having 3 to 5 carbon atoms,
and the salts of the compounds of formula (I) when R₁ represents a carboxylic acid functional group and the optical isomers of the said compounds of formula (I).

2. Compounds according to Claim 1, characterized by the fact that they exist in the form of salts of an alkali or alkaline-earth metal or alternatively of zinc or of an organic amine.

3. Compounds according to Claim 1, characterized by the fact that the lower alkyl radical has 1 to 6 carbon atoms and is chosen from the group consisting of the methyl, ethyl, isopropyl, butyl and tert-butyl radicals.

4. Compounds according to Claim 1, characterized by the fact that the alkoxy radical having 1 to 6 carbon atoms is chosen from the group consisting of a methoxy, ethoxy, isopropoxy and butoxy radical.

5. Compounds according to Claim 1, characterized by the fact that the α-substituted alkyl radical is chosen from the group consisting of: an isopropyl, 1-methylpropyl and 1-ethylpropyl radical.

6. Compounds according to Claim 1, characterized by the fact that the α,α'-disubstituted alkyl radical is chosen from the group consisting of: a tert-butyl, 1,1-dimethylpropyl, 1-methyl-1-ethylpropyl, 1-methyl-1-ethylhexyl and 1,1-dimethyldecyl radical.

7. Compounds according to Claim 1, characterized by the fact that the monohydroxyalkyl radical is a 2-hydroxyethyl, 2-hydroxypropyl or 3-hydroxypropyl radical.

8. Compounds according to Claim 1, characterized by the fact that the polyhydroxyalkyl radical contains 3 to 6 carbon atoms and 2 to 5 hydroxyl groups and is chosen from the group consisting of the 2,3-dihydroxypropyl, 2,3,4-trihydroxybutyl, 2,3,4,5-tetrahydroxypentyl radical and the pentaerythritol residue.

9. Compounds according to Claim 1, characterized by the fact that the aryl radical is a phenyl radical optionally substituted by at least one halogen atom, a hydroxyl or a nitro functional group.

10. Compounds according to Claim 1, characterized by the fact that the aralkyl radical is the benzyl or phenethyl radical optionally substituted by at least one halogen atom, a hydroxyl or a nitro functional group.

11. Compounds according to Claim 1, characterized by the fact that the mono- or polycyclic cycloalkyl radical having 5 to 12 carbon atoms of which the bonding carbon is trisubstituted, is the 1-methylcyclohexyl or 1-adamantyl radical.

12. Compounds according to Claim 1, characterized by the fact that the heterocycle is chosen from the group consisting of a piperidino, morpholino, pyrrolidino or piperazino radical, optionally substituted at position 4 by a C₁-C₆ alkyl radical or a mono- or polyhydroxyalkyl radical.

13. Compounds according to any one of the preceding claims, characterized by the fact that they are chosen from the group consisting of:
4-(5,6,7,8-tetrahydro-5,5,8,8-tetramethyl-2-naphthylglyoxyloyloxy)benzoic acid,
4-(5,6,7,8-tetrahydro-5,5,8,8-tetramethyl-2-naphthylacetoxy)benzoic acid,
4-(5,6,7,8-tetrahydro-5,5,8,8-tetramethyl-2-naphthylacetamido)benzoic acid,
4-(α-methylene-5,6,7,8-tetrahydro-5,5,8,8-tetramethyl-2-naphthylacetoxy)benzoic acid,
4-(5,6,7,8-tetrahydro-5,5,8,8-tetramethyl-2-naphthoyloxymethyl)benzoic acid,
4-(5,6,7,8-tetrahydro-5,5,8,8-tetramethyl-2-naphthoylthiomethyl)benzoic acid,
4-(5,6,7,8-tetrahydro-5,5,8,8-tetramethyl-2-naphtylcarboxamidomethyl)benzoic acid,
4-(N-methyl-5,6,7,8-tetrahydro-5,5,8,8-tetramethyl-2-naphthylcarboxamidomathyl)benzoic acid,
4-(5,6,7,8-tetrahydro-5,5,8,8-tetramethyl-2-naphthylmethyloxycarbonyl)benzoic acid,
4-[5,6,7,8-tetrahydro-5,5,8,8-tetramethyl-2-naphthyl(methylthio)carbonyl]benzoic acid,
5-(5,6,7,8-tetrahydro-5,5,8,8-tetramethyl-2-naphthoyloxymethyl)-2-thiophenecarboxylic acid,
4-(5,6,7,8-tetrahydro-5,5,8,8-tetramethyl-2-naphthyloxycarboxamido)benzoic acid,
4-(5,6,7,8-tetrahydro-5,5,8,8-tetramethyl-2-naphthylcarbonyldioxy)benzoic acid,
4-[1-(5,6,7,8-tetrahydro-5,5,8,8-tetramethyl-2-naphthoyloxy)ethyl]benzoic acid,
4-[[1-(5,6,7,8-tetrahydro-5,5,8,8-tetramethyl-2-naphthyl)ethyloxy]carbonyl]benzoic acid,
4-(3,5-di-tert-butyl-4-hydroxybenzoyloxymethyl)benzoic acid,
4-[3-(1-adamantyl)-4-methoxybenzoyloxymethyl]benzoic acid,
4-(3-tert-butyl-4-methoxybenzoyloxymethyl)benzoic acid,
4-(4-tert-butylbenzoyloxymethyl)benzoic acid,
4-[4-(1-adamantyl)-3-methoxybenzoyloxymethyl)]benzoic acid,
4-[3-(1-adamantyl)-4-methoxyphenoxycarboxamido]benzoic acid,
4-[3-(1-adamantyl)-4-methoxyphenylcarbonyldioxy]benzoic acid,
4-[3-(1-adamantyl)-4-methoxyphenylacetamido]benzoic acid,
4-(5,6,7,8-tetrahydro-5,5,8,8-tetramethyl-2-naphthoylformamido)benzoic acid,
4-(α-hydroxy-5,6,7,8-tetrahydro-5,5,8,8-tetramethyl-2-naphthylacetamido)benzoic acid,
4-(5,6,7,8-tetrahydro-5,5,8,8-tetramethyl-2-naphthylcarbamoylmethyl)benzoic acid,
2-hydroxy-4-(5,6,7,8-tetrahydro-5,5,8,8-tetramethyl-2-naphthylacetoxy)benzoic acid,
4-(5,6,7,8-tetrahydro-5,5,8,8-tetramethyl-2-naphthyloxycarbonylmethyl)benzoic acid,
4-(N-methyl-5,6,7,8-tetrahydro-5,5,8,8-tetramethyl-2-naphthylacetamido)benzoic acid,
4-(α-fluoro-5,6,7,8-tetrahydro-5,5,8,8-tetramethyl-2-naphthylacetamido)benzoic acid,
4-[3-(1-adamantyl)-4-methoxyphenylureido]benzoic acid,
2-hydroxy-4-(5,6,7,8-tetrahydro-5,5,8,8-tetramethyl-2-naphthoylmethyloxy)benzoic acid,
4-[2-(5,6,7,8-tetrahydro-5,5,8,8-tetrmethyl-2-naphthyl)propionamido]benzoic acid.

14. Pharmaceutical composition characterized by the fact that it contains in an appropriate vehicle, for enteral, parenteral, topical or ocular administration, at least one compound of formula (I) according to any one of Claims 1 to 13.

15. Composition according to Claim 14, characterized by the fact that it contains from 0.0001 to about 5 % by weight of a compound of formula (I).

16. Use of a compound according to any one of Claims 1 to 13 for the preparation of a pharmaceutical composition intended for the treatment of dermatological, rheumatic, respiratory as well as ophthalmological conditions.

17. Cosmetic composition for body and hair care, characterized by the fact that it contains, in an appropriate cosmetic vehicle, at least one compound of formula (I) according to any one of Claims 1 to 13.

18. Cosmetic composition according to Claim 17, characterized by the fact that it contains the compound of formula (I) at a concentration of between 0.0001 and 0.1 % and preferably between 0.001 and 0.01 % by weight.

## Patentansprüche

1. Biaromatische Verbindungen, dadurch gekennzeichnet, daß sie der folgenden allgemeinen Formel entsprechen: 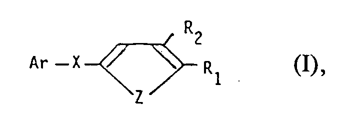 in der
Ar entweder einen Rest 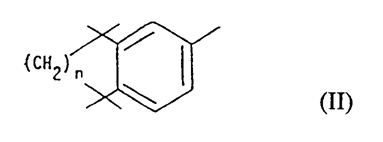 mit n gleich 1 oder 2
oder einen Rest 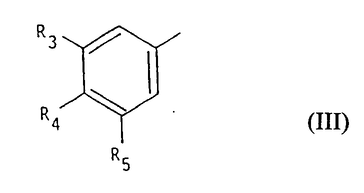 darstellt, worin
R₃ und R₅ ein Wasserstoffatom, eine OH-Gruppe, einen Alkoxyrest mit 1 bis 6 Kohlenstoffatomen, einen α-substituierten Alkylrest mit 3 bis 12 Kohlenstoffatomen oder einen α,α'-disubstituierten Alkylrest mit 4 bis 12 Kohlenstoffatomen, einen Cycloalkylrest mit 3 bis 12 Kohlenstoffatomen oder einen mono- oder polycyclischen Cycloalkylrest mit 5 bis 12 Kohlenstoffatomen, bei dem das verbindende Kohlenstoffatom trisubstituiert ist, bedeuten,
R₄ ein Wasserstoffatom, eine OH-Gruppe, einen Alkoxyrest mit 1 bis 6 Kohlenstoffatomen, einen α-substituierten Alkylrest mit 3 bis 12 Kohlenstoffatomen oder einen α,α'-disubstituierten Alkylrest mit 4 bis 12 Kohlenstoffatomen, einen Cycloalkylrest mit 3 bis 12 Kohlenstoffatomen oder einen mono- oder polycyclischen Cycloalkylrest mit 5 bis 12 Kohlenstoffatomen, bei dem das verbindende Kohlenstoffatom trisubstituiert ist, einen Monohydroxyalkylrest, einen Polyhydroxyalkylrest, ein Fluor- oder Chloratom, eine SH-Gruppe, einen Rest -SR₆, -SOR₆, -SO₂R₆, einen Alkenylrest mit 2 bis 6 Kohlenstoffatomen oder einen Alkenyloxyrest mit 2 bis 6 Kohlenstoffatomen darstellt, wobei
R₆ ein niedriger Alkylrest ist,
R₁ ein Wasserstoffatom, eine OH-Gruppe oder die Reste -CH₃, -CH₂OH, -COR₇, -CH(OH)CH₃, -CH₂OCOR₈, -SO₂R₉, -SOR₉ oder -SR₉ darstellt, worin
R₇ ein Wasserstoffatom, eine OH-Gruppe, den Rest -OR₁₀, den Rest -N(r'r''), einen niedrigen Alkylrest oder einen Monohydroxyalkyl-, Polyhydroxyalkyl- oder Zuckerrest darstellt, wobei
R₁₀ einen Alkylrest mit 1 bis 12 Kohlenstoffatomen oder einem Alkylenrest mit 2 bis 12 Kohlenstoffatomen und
r' und r'', die gleich oder verschieden sein können, ein Wasserstoffatom, einen niedrigen Alkyl-, Aryl- oder Aralkylrest, einen Aminosäure-, Zucker- oder Aminozuckerrest sowie einen Heterocyclus bedeuten oder r' und r'' zusammengenommen einen Heterocyclus bilden,
R₈ einen gesättigten oder ungesättigten, linearen oder verzweigten Alkylrest mit 1 bis 20 Kohlenstoffen oder einen Zuckerrest und
R₉ eine OH-Gruppe, einen niedrigen Alkylrest oder den Rest -N(r'r'') bedeuten,
R₂ ein Wasserstoffatom, eine OH-Gruppe, ein niedriger Alkylrest, ein Alkoxyrest mit 1 bis 6 Kohlenstoffatomen, ein Fluor- oder Chloratom, oder der Rest -CF₃, -COR₇, -CH₂OH oder CH₂OR₆ ist,
Z ein Sauerstoff- oder Schwefelatom oder ein divalenter Rest -CH=CR₁₁-, -N=CH- oder -N=CR₆- ist, wobei
R₁₁ ein Wasserstoffatom, eine OH-Gruppe, einen niedrigen Alkylrest, einen Alkoxyrest mit 1 bis 6 Kohlenstoffatomen, ein Fluor- oder Chloratom oder eine CF₃-Gruppe darstellt,
und X ein divalenter Rest ist, der von links nach rechts oder in der umgekehrten
Richtung gelesen werden kann und aus der folgenden Gruppe ausgewählt ist:
(i) 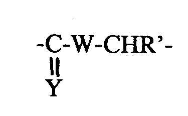 worin
R' ein Wasserstoffatom oder die CH₃-Gruppe,
W ein Sauerstoff- oder Schwefelatom oder der Rest -NR',
Y ein Sauerstoffatom oder bevorzugt ein Schwefelatom, wenn W der Rest -NR'-ist, bedeuten,
(ii) 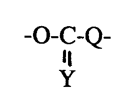 worin
Q ein Sauerstoffatom oder -NR'-,
Y ein Sauerstoffatom oder bevorzugt ein Schwefelatom, wenn W der Rest -NR'- ist, bedeuten,
(iii) 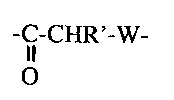
(iv) 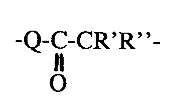 worin
R'' ein Wasserstoffatom, der Rest -CH₃, eine OH-Gruppe, ein Fluor- oder Chloratom ist oder R' und R'' zusammen einen Methylen- (=CH₂) oder Oxorest (=O) bilden,
(v) 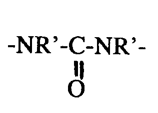 Y ein Sauerstoff oder Schwefelatom ist,
mit Ausnahme derjenigen Verbindungen, in denen X der Rest -C-CHR'-W-, Z der Rest -CH=CH- und R₂ Wasserstoff ist, wenn Ar einen Rest der Formel (III) darstellt, in der R₃ und R₅ verschieden sind und entweder Wasserstoffatome, einen α-substituierten oder einen α,α'-disubstituierten Alkylrest mit 3 bis 5 Kohlenstoffatomen darstellen,
sowie die Salze der Verbindungen der Formel (I), wenn R₁ eine Carboxylgruppe darstellt, und auch die optischen Isomere der Verbindungen der Formel (I).

2. Verbindungen gemäß Anspruch 1, dadurch gekennzeichnet, daß sie in Form eines Salzes mit einem Alkali- oder Erdalkalimetall oder auch mit Zink oder einem organischen Amin vorliegen.

3. Verbindungen gemäß Anspruch 1, dadurch gekennzeichnet, daß der niedrige Alkylrest 1 bis 6 Kohlenstoffatome aufweist und aus der aus Methyl, Ethyl, Isopropyl, Butyl oder tert.-Butyl bestehenden Gruppe ausgewählt ist.

4. Verbindungen gemäß Anspruch 1, dadurch gekennzeichnet, daß der Alkoxyrest mit 1 bis 6 Kohlenstoffatomen aus der aus Methoxy, Ethoxy, Isopropoxy und Butoxy bestehenden Gruppe ausgewählt ist.

5. Verbindungen gemäß Anspruch 1, dadurch gekennzeichnet, daß der α-substituierte Alkylrest aus der aus Isopropyl, 1-Methylpropyl und 1-Ethylpropyl bestehenden Gruppe ausgewählt ist.

6. Verbindungen gemäß Anspruch 1, dadurch gekennzeichnet, daß der α,α'-disubstituierte Alkylrest aus der aus tert.-Butyl, 1,1-Dimethylpropyl, 1-Methyl-1-ethylpropyl, 1-Methy-1-ethylhexyl und 1,1-Dimethyldecyl bestehenden Gruppe ausgewählt ist.

7. Verbindungen gemäß Anspruch 1, dadurch gekennzeichnet, daß der Monohydroxyalkylrest eine 2-Hydroxyethyl-, 2-Hydroxypropyl- oder 3-Hydroxypropylgruppe ist.

8. Verbindungen gemäß Anspruch 1, dadurch gekennzeichnet, daß der Polyhydroxyalkylrest 3 bis 6 Kohlenstoffatome und 2 bis 5 Hydroxylgruppen aufweist und aus der aus 2,3-Dihydroxypropyl, 2,3,4,5-Trihydroxybutyl, 2,3,4,5-Tetrahydroxypentyl oder Pentaerythrit bestehenden Gruppe ausgewählt ist.

9. Verbindungen gemäß Anspruch 1, dadurch gekennzeichnet, daß der Arylrest ein Phenylrest ist, welcher gegebenenfalls durch mindestens ein Halogenatom, eine Hydroxyl- oder eine Nitrogruppe substituiert ist.

10. Verbindungen gemäß Anspruch 1, dadurch gekennzeichnet, daß der Aralkylrest ein Benzyl- oder Phenylethylrest ist, welcher gegebenenfalls durch mindestens ein Halogenatom, eine Hydroxyl- oder eine Nitrogruppe substituiert ist.

11. Verbindung gemäß Anspruch 1, dadurch gekennzeichnet, daß der mono- oder polycyclische Alkylrest mit 5 bis 12 Kohlenstoffatomen, in dem das verbindende Kohlenstoffatom trisubstituiert ist, eine 1-Methylcyclohexyl- oder 1-Adamantylgruppe ist.

12. Verbindungen gemäß Anspruch 1, dadurch gekennzeichnet, daß der Heterocyclus aus der aus Piperidin, Morpholin, Pyrrolidin oder Piperazin bestehenden Gruppe ausgewählt ist, welche in Position 4 gegebenenfalls durch einen Alkylrest mit 1 bis 6 Kohlenstoffatomen oder einen Mono- oder Polyhydroxyalkylrest substituiert sein können.

13. Verbindungen gemäß einem der vorstehenden Ansprüche, dadurch gekennzeichnet, daß sie aus folgender Gruppe ausgewählt sind:
4-(5,6,7,8-Tetrahydro-5,5,8,8-tetramethyl-2-naphthylglyoxyloyloxy)-benzoesäure,
4-(5,6,7,8-Tetrahydro-5,5,8,8-tetramethyl-2-naphthylacetoxy)-benzoesäure,
4-(5,6,7,8-Tetrahydro-5,5,8,8-tetramethyl-2-naphthylacetamido)-benzoesäure,
4-(α-Methylen-5,6,7,8-tetrahydro-5,5,8,8-tetramethyl-2-naphthylacetoxy)benzoesäure,
4-(5,6,7,8-Tetrahydro-5,5,8,8-tetramethyl-2-naphthoyloxymethyl)-benzoesäure,
4-(5,6,7,8-Tetrahydro-5,5,8,8-tetramethyl-2-naphthoylthiomethyl)-benzoesäure,
4-(5,6,7,8-Tetrahydro-5,5,8,8-tetramethyl-2-naphthylcarboxamidomethyl)-benzoesäure,
4-(N-Methyl-5,6,7,8-tetrahydro-5,5,8,8-tetramethyl-2-naphthylcarboxamidomethyl)-benzoesäure,
4-(5,6,7,8-Tetrahydro-5,5,8,8-tetramethyl-2-naphthylmethyloxycarbonyl)-benzoesäure,
4-[5,6,7,8-Tetrahydro-5,5,8,8-tetramethyl-2-naphthyl-(methylthio)-carbonyl]-benzoesäure,
5-(5,6,7,8-Tetrahydro-5,5,8,8-tetramethyl-2-naphthoyloxymethyl)-2-thiophencarbonsäure,
4-(5,6,7,8-Tetrahydro-5,5,8,8-tetramethyl-2-naphthyloxycarboxamido)-benzoesäure,
4-(5,6,7,8-Tetrahydro-5,5,8,8-tetramethyl-2-naphthylcarbonyldioxy)-benzoesäure,
4-[1-(5,6,7,8-Tetrahydro-5,5,8,8-tetramethyl-2-naphthoyloxy)-ethyl]-benzoesäure,
4-[[1-(5,6,7,8-Tetrahydro-5,5,8,8-tetramethyl-2-naphthyl)-ethyloxy]-carbonyl]-benzoesäure,
4-(3,5-Di-tert.-butyl-4-hydroxybenzoyloxymethyl)-benzoesäure,
4-[3-(1-Adamantyl)-4-methoxybenzoyloxymethyl]-benzoesäure,
4-(3-tert.-Butyl-4-methoxybenzoyloxymethyl)benzoesäure,
4-(4-tert.-Butyl-4-benzoyloxymethyl)-benzoesäure,
4-[4-(1-Adamantyl)-3-methoxybenzoyloxymethyl]-benzoesäure,
4-[3-(1-Adamantyl)-4-methoxyphenoxycarboxamido]-benzoesäure,
4-[3-(1-Adamantyl)-4-methoxyphenylcarbonyldioxy]-benzoesäure,
4-[3-(1-Adamantyl)-4-methoxyphenylacetamido]-benzoesäure,
4-(5,6,7,8-Tetrahydro-5,5,8,8-tetramethyl-2-naphthoylformamido)-benzoesäure,
4-(α-Hydroxy-5,6,7,8-tetrahydro-5,5,8,8-tetramethyl-2-naphthylacetamido)-benzoesäure,
4-(5,6,7,8-Tetrahydro-5,5,8,8-tetramethyl-2-naphthylcarbamoylmethyl)-benzoesäure
2-Hydroxy-4-(5,6,7,8-tetrahydro-5,5,8,8-tetramethyl-2-naphthylacetoxy)-benzoesäure,
4-(5,6,7,8-Tetrahydro-5,5,8,8-tetramethyl-2-naphthyloxycarbonylmethyl)-benzoesäure,
4-(N-Methyl-5,6,7,8-tetrahydro-5,5,8,8-tetramethyl-2-naphthylacetamido)-benzoesäure,
4-(α-Fluor-5,6,7,8-tetrahydro-5,5,8,8-tetramethyl-2-naphthylacetamido)-benzoesäure,
4-[3-(1-Adamantyl)-4-methoxyphenylureido]-benzoesäure,
2-Hydroxy-4-(5,6,7,8-tetrahydro-5,5,8,8-tetramethyl-2-naphthoylmethyloxy)-benzoesäure und
4-[2-(5,6,7,8-Tetrahydro-5,5,8,8-tetramethyl-2-naphthyl)-propionamido]-benzoesäure.

14. Pharmazeutische Zubereitung, dadurch gekennzeichnet, daß sie in einem für die enterale, parenterale, topische oder ophtalmologische Applikation geeigneten Träger mindestens eine Verbindung der Formel (I) gemäß einem der vorstehenden Ansprüche 1 bis 13 enthält.

15. Zubereitung gemäß Anspruch 14, dadurch gekennzeichnet, daß sie von 0,0001 bis etwa 5 Gew.-% einer Verbindung der Formel (I) enthält.

16. Verwendung einer Verbindung gemäß einem der Ansprüche 1 bis 13 zur Herstellung einer pharmazeutischen Zubereitung zur Behandlung von dermatologischen oder rheumatischen Erkrankungen sowie von Erkrankungen des Respirationstraktes oder der Augen.

17. Kosmetische Zubereitung für die Körper- und Haarpflege, dadurch gekennzeichnet, daß sie in einem kosmetisch geeigneten Träger mindestens eine Verbindung der Formel (I) gemäß einem der Ansprüche 1 bis 13 enthält.

18. Kosmetische Zubereitung gemäß Anspruch 17, dadurch gekennzeichnet, daß sie die Verbindung der Formel (I) in einer Konzentration zwischen 0,0001 und 0,1 %, vorzugsweise zwischen 0,001 und 0,01 Gew.-% enthält.
